# EUROPEAN PATENT APPLICATION

(11) **EP 2 881 464 A1**
(43) Date of publication of application: **10.06.2015**
(21) Application number: 12877001.3
(22) Date of filing: 16.05.2012
(51) Int. Cl.: C12N 15/09, C12Q 1/68

(54) **METHOD FOR ASSESSING RISK OF IMPRINTING DISORDER**

(71) Applicant: Tohoku University, Aoba-ku Sendai-shi Miyagi 980-8577 (JP)
(72) Inventor: ARIMA, Takahiro, Sendai-shi Miyagi 980-8577 (JP)
(74) Representative: Wills, Andrew Jonathan
(86) International application number: PCT/JP2012/003196
(87) International publication number: WO 2013/171791

(57) **Abstract**

The present invention provides a method for assessing the risk that the progeny of a subject would develop an imprinting disorder, which comprises extracting genomic DNA from sperm collected from the subject, and measuring the methylation level of a CpG sequence comprised in the differentially methylation region of one or more maternally imprinted genes selected from the group consisting of DIRAS3, NAP1L5, FAM50B, GRB10, INPP5Fv2, RB1, ZNF597, ZNF331, PSIMCT-1, NNAT, L3MBTL, NESPAS, and GNAS1A, in the genomic DNA. According to the present invention, it becomes possible to safely and accurately assess the risk that the progeny of a subject would develop an imprinting disorder, using sperm collected from the subject.

## Description

### Technical Field

The present invention relates to a method for assessing the risk that the progeny of a subject would develop an imprinting disorder and a test kit for assessing the risk that the progeny of a subject would develop an imprinting disorder.

### Background Art

Assisted Reproductive Technology (ART), such as *in vitro* fertilization or micro fertilization, is an important treatment method for patients with infertility. However, in recent years, it has been reported that children who were born as a result of the ART would have a perinatal complication in newborn babies, such as congenital abnormality or low-birth-weight newborn, at a high frequency. In particular, it has been revealed that the frequency of development of imprinting disorders (imprinting diseases), such as Beckwith-Wiedemann syndrome (BWS) or Angelman syndrome (AS), is significantly increased as a result of the ART (Non-patent Document 1).

Genomic imprinting (genetic imprinting) is a phenomenon whereby allele-specific expression is observed in specific genes such that only a gene on one allele derived from one parent (father or mother) selectively functions and a gene on the other allele does not function. In the case of a gene that is subjected to genomic imprinting (imprinted gene), only one of two alleles (allelic genes), which is derived from a specific parent, is expressed. It has been made clear that such a uniparental expression of the imprinted gene is deeply associated with the methylation of cytosine in a CpG sequence comprised in a differentially methylation region (DMR). Since many DMRs are present in the promoter region, intron, and the like of an imprinted gene, it is considered that the imprinted gene is not expressed in an allele in which DMR is methylated, and that the imprinted gene is expressed in an allele in which DMR is not methylated.

The differentially methylated state of DMR of the imprinted gene is once reset in primordial germ cells (deletion of imprinting), and thereafter, the imprinting of each imprinted gene is established again in a sex-specific manner in the process of gametogenesis and/or maturation. A maternally imprinted gene (a gene in which only a mother-derived allele is methylated and inactivated) is methylated only in egg and is not methylated in sperm. On the other hand, a paternally imprinted gene (a gene in which only a father-derived allele is methylated and inactivated) is methylated only in sperm and is not methylated in egg. Imprinting (sex-specific methylated state) established in each of egg and sperm is stably maintained without receiving the influence of reprogramming (demethylation of the entire genome) that occurs at the initial stage of implantation after completion of the fertilization.

Studies have been actively conducted regarding imprinted genes, and many study papers have been reported. Lists of imprinted genes derived from various organisms are available from various academic websites, such as the website of the Mammalian Genetics Unit (MRC) (U.K.) (http://www.mgu.har.mrc.ac.uk/research/imprinting/imprin-viewdatagenes.html) or the website of University of Otago (New Zealand) (http://igc.otago.ac.nz/Search.html). For example, Non-patent Document 2 discloses that human ZDBF2 is one of paternally imprinted genes. Although there are only a limited number of reports regarding regulation of the methylation of imprinted genes during the process of gametogenesis and/or embryogenesis, it has been revealed that a maternally imprinted gene, SNRPN, is demethylated (Non-patent Document 3), and a paternally imprinted gene, H19, is methylated (Non-patent Documents 4 and 5), before these genes enter a meiosis process in a normal spermatogenesis process. Moreover, Non-patent Document 6 reports that the abnormal methylation of an imprinted gene in DMR is associated with various diseases including imprinting disorder as a typical example, abnormal development, malignant tumor, and the like.

Furthermore, as a result of previous studies, it has become clear that imprinted genes play an important role in the growth of newborn babies or the regulation of development. For instance, DMR, which exists from the promoter to exon 1 and intron 1 of a PEG1 gene in humans and mice, is not generally methylated in a paternal allele. It has been reported that if such a paternal allele is methylated and inactivated, it would cause an increase in the growth retardation or death of a fetus. Further, Non-patent Document 5 states that the imprinting abnormality of H19 is observed in the sperm of patients with oligozoospermia or a limited number of patients with infertility who have normal sperm. However, there is also a report stating that the methylation of an imprinted gene in the sperm of a patient with male infertility has been normal (Non-patent Document 7), and further, there is another report stating that excessive induction of ovulation in ART has an influence on the methylation of specific imprinted genes (Non-patent Document 8). Hence, the correlation of imprinted genes and male infertility has not yet been elucidated sufficiently.

Patent Document 1 discloses a method of detecting abnormal methylation in the DMR of maternally imprinted genes (PEG1, LIT1, ZAC, PEG3 and SNRPN) and paternally imprinted gene (H19 and GTL2) in the sperm of a patient with infertility, so as to determine the risk of developing imprinting disorder in the aforementioned sperm. In addition, Patent Document 2 discloses a method for examining imprinting disorder, which comprises detecting abnormal methylation in a paternally imprinted gene ZDBF2 in the sperm of a patient with infertility or cancer tissues. However, there are still many unclear points regarding the mechanism of developing imprinting disorder, and it is considered that many other genes would be associated with imprinting disorder.

### Prior Art Documents

### Patent Documents

Patent Document 1: Japanese unexamined Patent Application Publication No. 2009-165409
Patent Document 2: Japanese unexamined Patent Application Publication No. 2011-239750

### Non-patent Documents

Non-patent Document 1: Ogata T. and Kagami M. J. Mamm. Ova. Res. Vol. (2006) 23, 158-162
Non-patent Document 2: Kobayashi H., et al., Genomics (2009) 93: 461-472
Non-patent Document 3: Manning M., et al, Urol. Int. (2001) 67: 151-155
Non-patent Document 4: Kerjean A., et al., Hum. Mol. Genet. (2000) 9: 2183-2187
Non-patent Document 5: Marques C. J., et al., Lancet (2004) 363: 1700-1702
Non-patent Document 6: Paulsen M. and Ferguson-Smith A. C., J. Pathol. (2001) 195: 97-110
Non-patent Document 7: Hartmann S., et al., Mol. Hum. Reprod. (2006) 12: 407-411
Non-patent Document 8: Sato A., et al., Hum. Reprod. (2007) 22: 26-35

### Summary of the Invention

### Object to be Solved by the Invention

It is an object of the present invention to identify a novel differentially methylation region associated with the development of an imprinting disorder, and to provide a method for assessing the risk that the progeny of a subject would develop an imprinting disorder, in which the methylation level of the aforementioned differentially methylation region in the genomic DNA of the sperm of a subject is used as an indicator.

### Means to Solve the Object

The present inventor has first extracted genomic DNA from sperm and blood derived from 20 normal individuals, and has analyzed the methylated state of the differentially methylation region of each of DIRAS3, NAP1L5, FAM50B, GRB10, INPP5Fv2, RB1, ZNF597, ZNF331, PSIMCT-1, NNAT, L3MBTL, NESPAS, and GNAS1A. As a result, it was found that almost 100% methylated alleles and almost 100% demethylated alleles are present at a ratio of 1:1 in all of the differentially methylation regions in the case of the genomic DNA of blood, and that highly methylated alleles are not present and almost 100% demethylated alleles are only present therein in the case of the genomic DNA of sperm. From these results, it was demonstrated that DIRAS3, NAP1L5, FAM50B, GRB10, INPP5Fv2, RB1, ZNF597, ZNF331, PSIMCT-1, NNAT, L3MBTL, NESPAS, and GNAS1A are maternally imprinted genes in which only the paternal allele genes function and the maternal allele genes do not function, in normal individuals.

Moreover, from the aforementioned results, it is considered that if the differentially methylation region of the aforementioned maternally imprinted genes is highly methylated in the genomic DNA of sperm, a child derived from the sperm would develop an imprinting disorder, since the paternal allele genes, which should originally function, cannot function. Thus, in order to examine the correlation of abnormal methylation in the differentially methylation region of the aforementioned maternally imprinted genes and an imprinting disorder, the present inventor has extracted genomic DNA from blood derived from patients with Silver-Russell syndrome (SRS) and patients with Beckwith-Wiedemann syndrome (BWS), and has then analyzed the methylated state of the differentially methylation region of each of DIRAS3, NAP1L5, FAM50B, GRB10, INPP5Fv2, RB1, ZNF597, ZNF331, PSIMCT-1, NNAT, L3MBTL, NESPAS, and GNAS1A. As a consequence, it became clear that the abnormal methylation of GRB10, ZNF597, INPP5Fv2, ZNF331, and FAM50B was observed in SRS patients, and also that the abnormal methylation of ZNF331 was observed in BWS patients. From these results, it was strongly suggested that abnormal methylation in the differentially methylation region of the aforementioned maternally imprinted genes would be associated with the development of an imprinting disorder.

As described above, the present inventor has found for the first time that DIRAS3, NAP1L5, FAM50B, GRB10, INPP5Fv2, RB1, ZNF597, ZNF331, PSIMCT-1, NNAT, L3MBTL, NESPAS, and GNAS1A are maternally imprinted genes, and that abnormal methylation in the differentially methylation region of each of these genes is associated with the development of an imprinting disorder, thereby completing the present invention.

Specifically, the present invention relates to
[1] a method for assessing the risk that the progeny of a subject would develop an imprinting disorder, which comprises the following steps (a) and (b):
   (a) a step of extracting genomic DNA from sperm collected from the subject; and
   (b) a step of measuring the methylation level of cytosine residues in a CpG sequence comprised in the differentially methylation region of one or more maternally imprinted genes selected from the group consisting of DIRAS3, NAP1L5, FAM50B, GRB10, INPP5Fv2, RB1, ZNF597, ZNF331, PSIMCT-1, NNAT, L3MBTL, NESPAS, and GNAS1A, in the genomic DNA extracted in the step (a);
[2] the method according to the above [1], wherein the differentially methylation region of DIRAS3 is a region at positions 1849-2197 (SEQ ID NO: 1) in the nucleotide sequence of human chromosome 1 registered under GenBank Accession No. AF202543.1 (date of update: August 13, 2001);
[3] the method according to the above [1] or [2], wherein the CpG sequence comprised in the differentially methylation region of DIRAS3 is one or more CpG sequences selected from the CpG sequences at positions 1873-1874, 1893-1894, 1900-1901, 1902-1903, 1905-1906, 1912-1913, 1947-1948, 1954-1955, 1960-1961, 1991-1992, 1997-1998, 2004-2005, 2014-2015, 2021-2022, 2034-2035, 2036-2037, 2072-2073, 2074-2075, 2086-2087, 2093-2094, 2120-2121, 2128-2129, and 2150-2151, in the nucleotide sequence of human chromosome 1 registered under GenBank Accession No. AF202543.1 (date of update: August 13, 2001);
[4] the method according to any one of the above [1] to [3], wherein the differentially methylation region of NAP1L5 is a region at positions 35225-35572 (SEQ ID NO: 2) in the nucleotide sequence of human chromosome 4 registered under GenBank Accession No. AC108065.3 (date of update: May 24, 2002);
[5] the method according to any one of the above [1] to [4], wherein the CpG sequence comprised in the differentially methylation region of NAP1L5 is one or more CpG sequences selected from the CpG sequences at positions 35258-35259, 35260-35261, 35279-35280, 35281-35282, 35303-35304, 35308-35309, 35315-35316, 35333-35334,35342-35343,35345-35346, 35354-35355, 35369-35370, 35387-35388, 35392-35393, 35413-35414, 35435-35436, 35452-35453, 35475-35476, 35481-35482, 35491-35492, 35497-35498, 35501-35502, 35505-35506, 35507-35508, 35516-35517, 35523-35524, 35525-35526, 35531-35532, 35544-35545, and 35546-35547, in the nucleotide sequence of human chromosome 4 registered under GenBank Accession No. AC108065.3 (date of update: May 24, 2002);
[6] the method according to any one of the above [1] to [4], wherein, in the differentially methylation region of NAP1L5, when the thymine residue at position 35330 in the nucleotide sequence of human chromosome 4 registered under GenBank Accession No. AC108065.3 (date of update: May 24, 2002) is mutated to an cytosine residue, the CpG sequence comprised in the differentially methylation region of NAP1L5 is one or more CpG sequences selected from the CpG sequences at positions 35258-35259, 35260-35261, 35279-35280, 35281-35282, 35303-35304, 35308-35309, 35315-35316, 35329-35330, 35333-35334, 35342-35343, 35345-35346, 35354-35355, 35369-35370, 35387-35388, 35392-35393, 35413-35414, 35435-35436, 35452-35453, 35475-35476, 35481-35482, 35491-35492, 35497-35498, 35501-35502, 35505-35506, 35507-35508, 35516-35517, 35523-35524, 35525-35526, 35531-35532, 35544-35545, and 35546-35547 in the nucleotide sequence of human chromosome 4 registered under GenBank Accession No. AC108065.3 (date of update: May 24, 2002);
[7] the method according to any one of the above [1] to [6], wherein the differentially methylation region of FAM50B is a region at positions 350-683 (SEQ ID NO: 3) in the nucleotide sequence of human chromosome 6 registered under GenBank Accession No. Y18504.1 (date of update: November 14, 2006);
[8] the method according to any one of the above [1] to [7], wherein the CpG sequence comprised in the differentially methylation region of FAM50B is one or more CpG sequences selected from the CpG sequences at positions 379-380, 384-385, 401-402, 419-420, 434-435, 438-439, 457-458, 471-472, 488-489, 498-499, 518-519, 539-540, 541-542, 549-550, 565-566, 582-583, 601-602, 609-610, 619-620, 643-644, and 649-650, in the nucleotide sequence of human chromosome 6 registered under GenBank Accession No. Y18504.1 (date of update: November 14, 2006);
[9] the method according to any one of the above [1] to [8], wherein the differentially methylation region of GRB10 is a region at positions 42226-42470 (SEQ ID NO: 4) in the nucleotide sequence of human chromosome 7 registered under GenBank Accession No. AC004920.2 (date of update: October 15, 2003);
[10] the method according to any one of the above [1] to [9], wherein the CpG sequence comprised in the differentially methylation region of GRB10 is one or more CpG sequences selected from the CpG sequences at positions 42251-42252, 42256-42257, 42264-42265, 42273-42274, 42275-42276, 42277-42278, 42284-42285, 42288-42289, 42290-42291, 42298-42299, 42305-42306, 42313-42314, 42316-42317, 42328-42329, 42335-42336, 42339-42340, 42353-42354, 42355-42356, 42361-42362, 42365-42366, 42369-42370, 42375-42376, 42388-42389, 42399-42400, 42409-42410, 42421-42422, 42423-42424, 42429-42430, 42436-42437, and 42440-42441, in the nucleotide sequence of human chromosome 7 registered under GenBank Accession No. AC004920.2 (date of update: October 15, 2003);
[11] the method according to any one of the above [1] to [6], wherein the differentially methylation region of INPP5Fv2 is a region at positions 22533-22735 (SEQ ID NO: 5) in the nucleotide sequence of human chromosome 10 registered under GenBank Accession No. AL133461.10 (date of update: January 13, 2009);
[12] the method according to any one of the above [1] to [11], wherein the CpG sequence comprised in the differentially methylation region of INPP5Fv2 is one or more CpG sequences selected from the CpG sequences at positions 22556-22557, 22564-22565, 22567-22568, 22571-22572, 22579-22580, 22583-22584, 22595-22596, 22601-22602, 22603-22604, 22619-22620, 22626-22627, 22628-22629, 22633-22634, 22647-22648, 22656-22657, and 22674-22675, in the nucleotide sequence of human chromosome 10 registered under GenBank Accession No. AL133461.10 (date of update: January 13, 2009);
[13] the method according to any one of the above [1] to [12], wherein the differentially methylation region of RB1 is a region at positions 30347-30587 (SEQ ID NO: 6) in the nucleotide sequence of human chromosome 13 registered under GenBank Accession No. AL392048.9 (date of update: January 13, 2009);
[14] the method according to any one of the above [1] to [13], wherein the CpG sequence comprised in the differentially methylation region of RB1 is one or more CpG sequences selected from the CpG sequences at positions 30370-30371, 30403-30404, 30426-30427, 30443-30444, 30452-30453, 30455-30456, 30464-30465, 30476-30477, 30479-30480, 30487-30488, 30497-30498, 30514-30515, and 30558-30559, in the nucleotide sequence of human chromosome 13 registered under GenBank Accession No. AL392048.9 (date of update: January 13, 2009);
[15] the method according to any one of the above [1] to [14], wherein the differentially methylation region of ZNF597 is a region at positions 140956-141209 (SEQ ID NO: 7) in the nucleotide sequence of human chromosome 16 registered under GenBank Accession No. AC025283.6 (date of update: September 5, 2002);
[16] the method according to any one of the above [1] to [15], wherein the CpG sequence comprised in the differentially methylation region of ZNF597 is one or more CpG sequences selected from the CpG sequences at positions 140981-140982, 140984-140985, 140988-140989, 140992-140993, 140997-140998, 140999-141000, 141002-141003, 141009-141010, 141014-141015, 141034-141035, 141052-141053, 141063-141064, 141073-141074, 141076-141077, 141082-141083, 141087-141088, 141103-141104, 141126-141127, 141137-141138, and 141183-141184, in the nucleotide sequence of human chromosome 16 registered under GenBank Accession No. AC025283.6 (date of update: September 5, 2002);
[17] the method according to any one of the above [1] to [15], wherein, in the differentially methylation region of ZNF597, when the guanine residue at position 141064 in the nucleotide sequence of human chromosome 16 registered under GenBank Accession No. AC025283.6 (date of update: September 5, 2002) is mutated to an adenine residue, the CpG sequence comprised in the differentially methylation region of ZNF597 is one or more CpG sequences selected from the CpG sequences at positions 140981-140982, 140984-140985, 140988-140989, 140992-140993, 140997-140998, 140999-141000, 141002-141003, 141009-141010, 141014-141015, 141034-141035, 141052-141053, 141073-141074, 141076-141077, 141082-141083, 141087-141088, 141103-141104, 141126-141127, 141137-141138, and 141183-141184 in the nucleotide sequence of human chromosome 16 registered under GenBank Accession No. AC025283.6 (date of update: September 5, 2002);
[18] the method according to any one of the above [1] to [17], wherein the differentially methylation region of ZNF331 is a region at positions 106090-106360 (SEQ ID NO: 8) in the nucleotide sequence of human chromosome 19 registered under GenBank Accession No. AC011487.5 (date of update: February 28, 2001); and
[19] the method according to any one of the above [1] to [18], wherein the CpG sequence comprised in the differentially methylation region of ZNF331 is one or more CpG sequences selected from the CpG sequences at positions 106115-106116, 106117-106118, 106123-106124, 106127-106128, 106129-106130, 106138-106139, 106142-106143, 106147-106148, 106149-106150, 106168-106169, 106173-106174, 106197-106198, 106207-106208, 106220-106221, 106225-106226, 106235-106236, 106249-106250, 106259-106260, 106275-106276, 106285-106286, 106303-106304, 106307-106308, 106313-106314, 106317-106318, 106328-106329, and 106333-106334, in the nucleotide sequence of human chromosome 19 registered under GenBank Accession No. AC011487.5 (date of update: February 28, 2001).

Moreover, the present invention relates to [20] the method according to any one of the above [1] to [18], wherein, in the differentially methylation region of ZNF331, when the cytosine residue at position 106235 in the nucleotide sequence of human chromosome 19 registered under GenBank Accession No. AC011487.5 (date of update: February 28, 2001) is mutated to a thymine residue, the CpG sequence comprised in the differentially methylation region of ZNF331 is one or more CpG sequences selected from the CpG sequences at positions 106115-106116, 106117-106118, 106123-106124, 106127-106128, 106129-106130, 106138-106139, 106142-106143, 106147-106148, 106149-106150, 106168-106169, 106173-106174, 106197-106198, 106207-106208, 106220-106221, 106225-106226, 106249-106250, 106259-106260, 106275-106276, 106285-106286, 106303-106304, 106307-106308, 106313-106314, 106317-106318, 106328-106329, and 106333-106334 in the nucleotide sequence of human chromosome 19 registered under GenBank Accession No. AC011487.5 (date of update: February 28, 2001);
the method according to any one of the above [1] to [20], wherein the differentially methylation region of PSIMCT-1 is a region at positions 20820-21147 (SEQ ID NO: 9) in the nucleotide sequence of human chromosome 20 registered under GenBank Accession No. AL110115.38 (date of update: January 13, 2009);
the method according to any one of the above [1] to [21], wherein the CpG sequence comprised in the differentially methylation region of PSIMCT-1 is one or more CpG sequences selected from the CpG sequences at positions 20844-20845, 20872-20873, 20883-20884, 20892-20893, 20898-20899, 20903-20904, 20908-20909, 20919-20920, 20939-20940, 20944-20945, 20951-20952, 20953-20954, 20972-20973, 20979-20980, 20985-20986, 20995-20996, 21007-21008, 21009-21010, 21014-21015, 21018-21019, 21020-21021, 21023-21024, 21047-21048, 21064-21065, 21082-21083, and 21086-21087, in the nucleotide sequence of human chromosome 20 registered under GenBank Accession No. AL110115.38 (date of update: January 13, 2009);
the method according to any one of the above [1] to [22], wherein the differentially methylation region of NNAT is a region at positions 61633-61902 (SEQ ID NO: 10) in the nucleotide sequence of human chromosome 20 registered under GenBank Accession No. AL109614.28 (date of update: January 13, 2009);
the method according to any one of claims [1] to [23], wherein the CpG sequence comprised in the differentially methylation region of NNAT is one or more CpG sequences selected from the CpG sequences at positions 61659-61660, 61666-61667, 61708-61709, 61719-61720, 61757-61758, 61759-61760, 61765-61766, 61778-61779, 61782-61783, 61795-61796, 61797-61798, 61804-61805, 61806-61807, 61812-61813, 61820-61821, 61830-61831, 61837-61838, 61846-61847, 61853-61854, and 61870-61871, in the nucleotide sequence of human chromosome 20 registered under GenBank Accession No. AL109614.28 (date of update: January 13, 2009);
the method according to any one of the above [1] to [24], wherein the differentially methylation region of L3MBTL is a region at positions 161428-161758 (SEQ ID NO: 11) in the nucleotide sequence of human chromosome 20 registered under GenBank Accession No. AL031681.16 (date of update: January 13, 2009);
the method according to any one of the above [1] to [25], wherein the CpG sequence comprised in the differentially methylation region of L3MBTL is one or more CpG sequences selected from the CpG sequences at positions 161454-161455, 161480-161481, 161497-161498, 161517-161518, 161523-161524, 161541-161542, 161545-161546, 161552-161553, 161571-161572, 161573-161574, 161584-161585, 161592-161593, 161603-161604, 161615-161616, 161633-161634, 161640-161641, 161647-161648, 161658-161659, 161664-161665, 161670-161671, 161679-161680, 161687-161688, 161690-161691, 161700-161701, 161705-161706, 161720-161721, and 161733-161734, in the nucleotide sequence of human chromosome 20 registered under GenBank Accession No. AL031681.16 (date of update: January 13, 2009);
the method according to any one of the above [1] to [26], wherein the differentially methylation region of NESPAS is a region at positions 12577-12888 (SEQ ID NO: 12) in the nucleotide sequence of human chromosome 20 registered under GenBank Accession No. AJ251760.1 (date of update: November 14, 2006);
the method according to any one of the above [1] to [27], wherein the CpG sequence comprised in the differentially methylation region of NESPAS is one or more CpG sequences selected from the CpG sequences at positions 12615-12616, 12620-12621, 12624-12625, 12631-12632, 12639-12640, 12646-12647, 12659-12660, 12666-12667, 12668-12669, 12670-12671, 12699-12700, 12706-12707, 12719-12720, 12735-12736, 12792-12793, 12806-12807, 12828-12829, 12835-12836, 12859-12860, and 12862-12863, in the nucleotide sequence of human chromosome 20 registered under GenBank Accession No. AJ251760.1 (date of update: November 14, 2006);
the method according to any one of the above [1] to [28], wherein the differentially methylation region of GNAS1A is a region at positions 1698-1933 (SEQ ID NO: 13) in the nucleotide sequence of human chromosome 20 registered under GenBank Accession No. AF246983.1 (date of update: November 20, 2000);
the method according to any one of the above [1] to [29], wherein the CpG sequence comprised in the differentially methylation region of GNAS1A is one or more CpG sequences selected from the CpG sequences at positions 1727-1728, 1734-1735, 1737-1738, 1747-1748, 1749-1750, 1757-1758, 1762-1763, 1766-1767, 1769-1770, 1782-1783, 1785-1786, 1791-1792, 1807-1808, 1811-1812, 1815-1816, 1818-1819, 1831-1832, 1836-1837, 1842-1843, 1883-1884, and 1908-1909, in the nucleotide sequence of human chromosome 20 registered under GenBank Accession No. AF246983.1 (date of update: November 20, 2000);
the method according to any one of the above [1] to [30], wherein the imprinting disorder is Russell-Silver syndrome or Beckwith-Wiedemann syndrome;
the method according to any one of the above [1] to [31], wherein the subject is a patient with male infertility;
the method according to any one of the above [1] to [32], wherein, in the step (b), the methylation level is measured by a bisulfite sequence method;
the method according to the above [33], wherein the bisulfite sequence method uses one or more primer sets selected from the following i) to xiii):
i) a primer set for amplifying the differentially methylation region of DIRAS3, which consists of the nucleotide sequences shown in SEQ ID NOS: 14 and 15;
ii) a primer set for amplifying the differentially methylation region of NAP1L5, which consists of the nucleotide sequences shown in SEQ ID NOS: 16 and 17;
iii) a primer set for amplifying the differentially methylation region of FAM50B, which consists of the nucleotide sequences shown in SEQ ID NOS: 18 and 19;
iv) a primer set for amplifying the differentially methylation region of GRB10, which consists of the nucleotide sequences shown in SEQ ID NOS: 20 and 21;
v) a primer set for amplifying the differentially methylation region of INPP5Fv2, which consists of the nucleotide sequences shown in SEQ ID NOS: 22 and 23;
vi) a primer set for amplifying the differentially methylation region of RB1, which consists of the nucleotide sequences shown in SEQ ID NOS: 24 and 25;
vii) a primer set for amplifying the differentially methylation region of ZNF597, which consists of the nucleotide sequences shown in SEQ ID NOS: 26 and 27;
viii) a primer set for amplifying the differentially methylation region of ZNF331, which consists of the nucleotide sequences shown in SEQ ID NOS: 28 and 29;
ix) a primer set for amplifying the differentially methylation region of PSIMCT-1, which consists of the nucleotide sequences shown in SEQ ID NOS: 30 and 31;
x) a primer set for amplifying the differentially methylation region of NNAT, which consists of the nucleotide sequences shown in SEQ ID NOS: 32 and 33;
xi) a primer set for amplifying the differentially methylation region of L3MBTL, which consists of the nucleotide sequences shown in SEQ ID NOS: 34 and 35;
xii) a primer set for amplifying the differentially methylation region of NESPAS, which consists of the nucleotide sequences shown in SEQ ID NOS: 36 and 37; and
xiii) a primer set for amplifying the differentially methylation region of GNAS1A, which consists of the nucleotide sequences shown in SEQ ID NOS: 38 and 39;
a test kit for assessing the risk that the progeny of a subject would develop an imprinting disorder, wherein the test kit comprises one or more primer sets selected from the following i) to xiii):
i) a primer set for amplifying the differentially methylation region of DIRAS3, which consists of the nucleotide sequences shown in SEQ ID NOS: 14 and 15;
ii) a primer set for amplifying the differentially methylation region of NAP1L5, which consists of the nucleotide sequences shown in SEQ ID NOS: 16 and 17;
iii) a primer set for amplifying the differentially methylation region of FAM50B, which consists of the nucleotide sequences shown in SEQ ID NOS: 18 and 19;
iv) a primer set for amplifying the differentially methylation region of GRB10, which consists of the nucleotide sequences shown in SEQ ID NOS: 20 and 21;
v) a primer set for amplifying the differentially methylation region of INPP5Fv2, which consists of the nucleotide sequences shown in SEQ ID NOS: 22 and 23;
vi) a primer set for amplifying the differentially methylation region of RB1, which consists of the nucleotide sequences shown in SEQ ID NOS: 24 and 25;
vii) a primer set for amplifying the differentially methylation region of ZNF597, which consists of the nucleotide sequences shown in SEQ ID NOS: 26 and 27;
viii) a primer set for amplifying the differentially methylation region of ZNF331, which consists of the nucleotide sequences shown in SEQ ID NOS: 28 and 29;
ix) a primer set for amplifying the differentially methylation region of PSIMCT-1, which consists of the nucleotide sequences shown in SEQ ID NOS: 30 and 31;
x) a primer set for amplifying the differentially methylation region of NNAT, which consists of the nucleotide sequences shown in SEQ ID NOS: 32 and 33;
xi) a primer set for amplifying the differentially methylation region of L3MBTL, which consists of the nucleotide sequences shown in SEQ ID NOS: 34 and 35;
xii) a primer set for amplifying the differentially methylation region of NESPAS, which consists of the nucleotide sequences shown in SEQ ID NOS: 36 and 37; and
xiii) a primer set for amplifying the differentially methylation region of GNAS1A, which consists of the nucleotide sequences shown in SEQ ID NOS: 38 and 39.

### Effect of the Invention

According to the present invention, it becomes possible to safely and accurately assess the risk that the progeny of a subject would develop an imprinting disorder, using sperm collected from the subject.

### Brief Description of Drawings

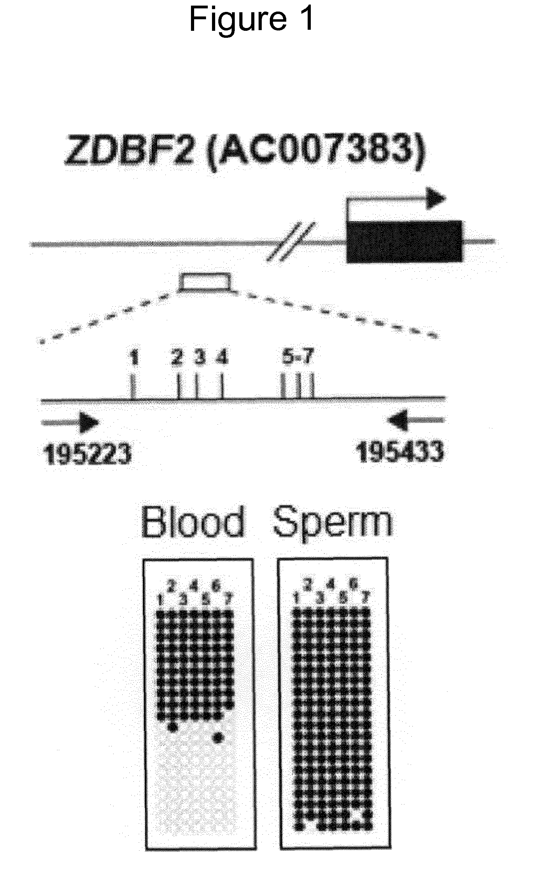

[Figure 1] Figure 1 is a view showing the results obtained by analyzing the methylated state of the differentially methylation region of ZDBF2 and the methylated state of the differentially methylation region in genomic DNA derived from blood and sperm collected from normal individuals. In the figure, the symbol "○" indicates a non-methylated CpG sequence, and the symbol "●" indicates a methylated CpG sequence.

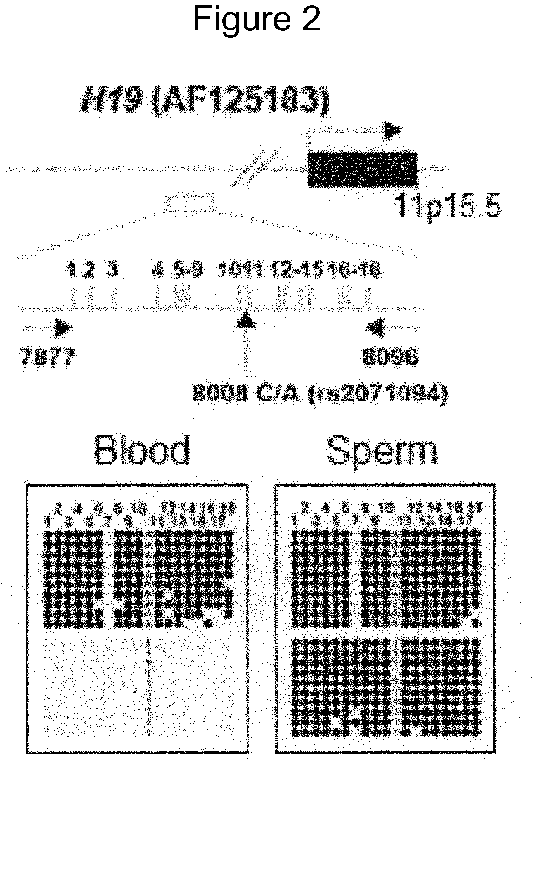

[Figure 2] Figure 2 is a view showing the results obtained by analyzing the methylated state of the differentially methylation region of H19 and the methylated state of the differentially methylation region in genomic DNA derived from blood and sperm collected from normal individuals. In the upper figure, the symbol "C/A" indicates a single nucleotide polymorphism site, and in the lower figure, the symbol "○" indicates a non-methylated CpG sequence and the symbol "●" indicates a methylated CpG sequence.

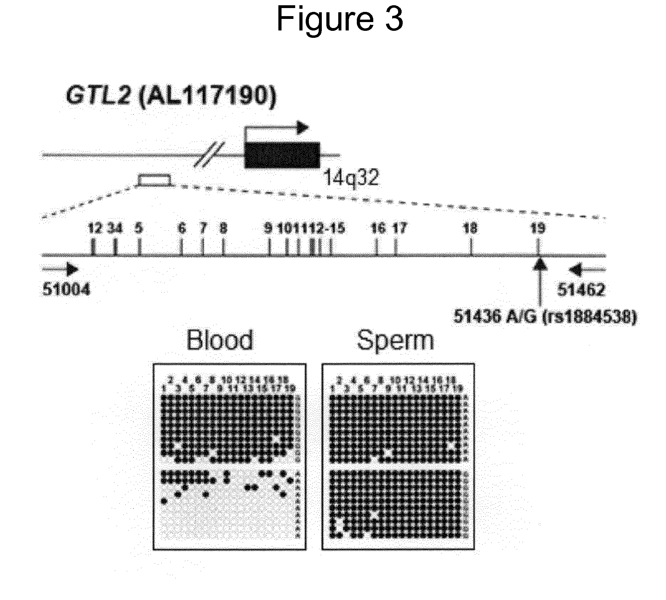

[Figure 3] Figure 3 is a view showing the results obtained by analyzing the methylated state of the differentially methylation region of GTL2 and the methylated state of the differentially methylation region in genomic DNA derived from blood and sperm collected from normal individuals. In the upper figure, the symbol "A/G" indicates a single nucleotide polymorphism site, and in the lower figure, the symbol "○" indicates a non-methylated CpG sequence and the symbol "●" indicates a methylated CpG sequence.

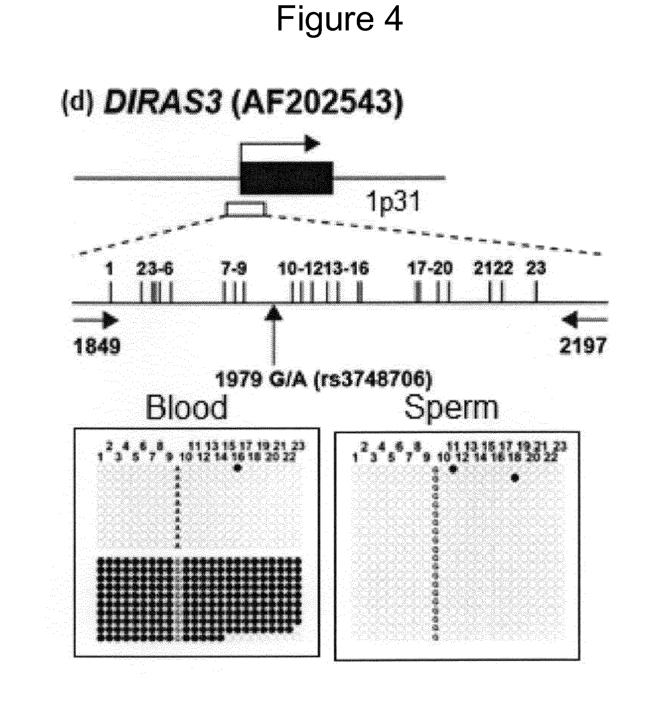

[Figure 4] Figure 4 is a view showing the results obtained by analyzing the methylated state of the differentially methylation region of DIRAS3 and the methylated state of the differentially methylation region in genomic DNA derived from blood and sperm collected from normal individuals. In the upper figure, the symbol "G/A" indicates a single nucleotide polymorphism site. Since Figure 4 shows the analysis of a sequence complementary to a DNA sequence corresponding to positions 1849-2197 of GenBank Accession No. AF202543.1, a single nucleotide polymorphism of "C/T" at position 1979 is indicated as "G/A." In addition, in the lower figure, the symbol "○" indicates a non-methylated CpG sequence, and the symbol "●" indicates a methylated CpG sequence.

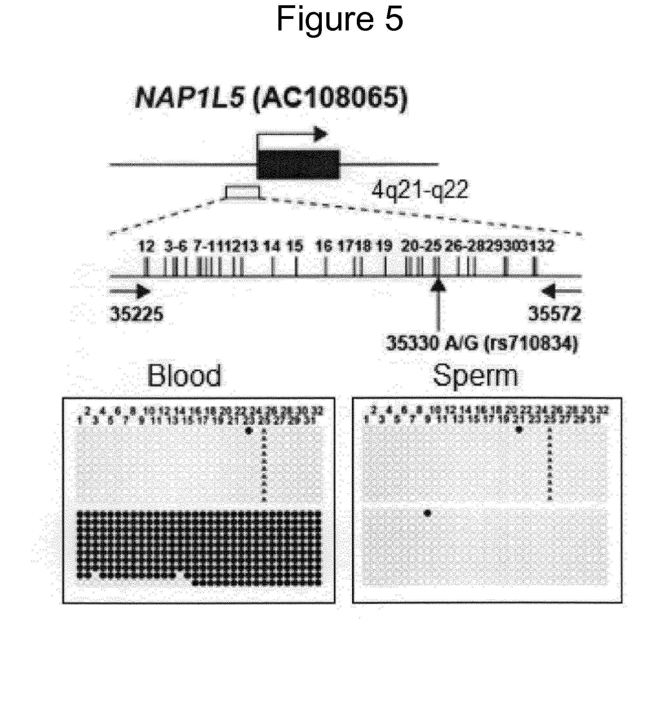

[Figure 5] Figure 5 is a view showing the results obtained by analyzing the methylated state of the differentially methylation region of NAP1L5 and the methylated state of the differentially methylation region in genomic DNA derived from blood and sperm collected from normal individuals. In the upper figure, the symbol "A/G" indicates a single nucleotide polymorphism site. Since Figure 5 shows the analysis of a sequence complementary to a DNA sequence corresponding to positions 35225-35572 of GenBank Accession No. AC108065.3, a single nucleotide polymorphism of "T/C" at position 35330 is indicated as "A/G." In addition, in the lower figure, the symbol "○" indicates a non-methylated CpG sequence, and the symbol "●" indicates a methylated CpG sequence.

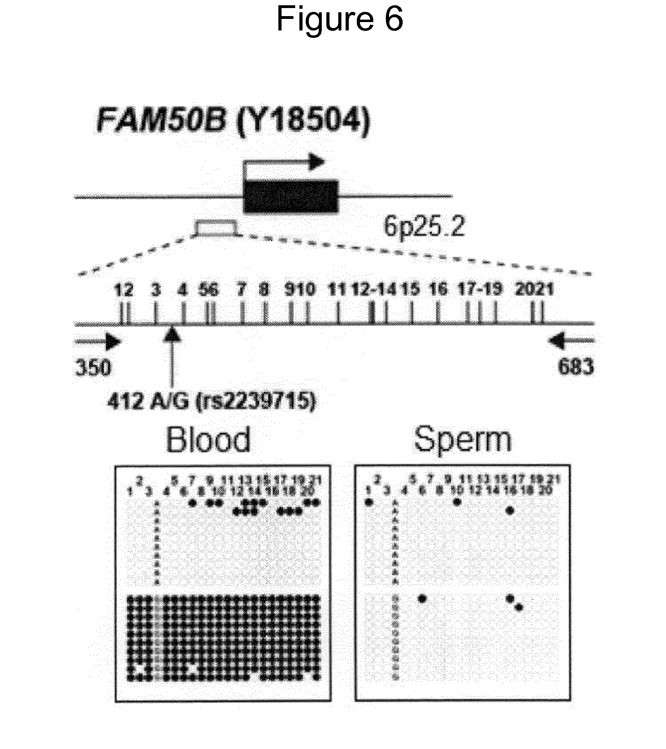

[Figure 6] Figure 6 is a view showing the results obtained by analyzing the methylated state of the differentially methylation region of FAM50B and the methylated state of the differentially methylation region in genomic DNA derived from blood and sperm collected from normal individuals. In the upper figure, the symbol "A/G" indicates a single nucleotide polymorphism site. In addition, in the lower figure, the symbol "○" indicates a non-methylated CpG sequence, and the symbol "●" indicates a methylated CpG sequence.

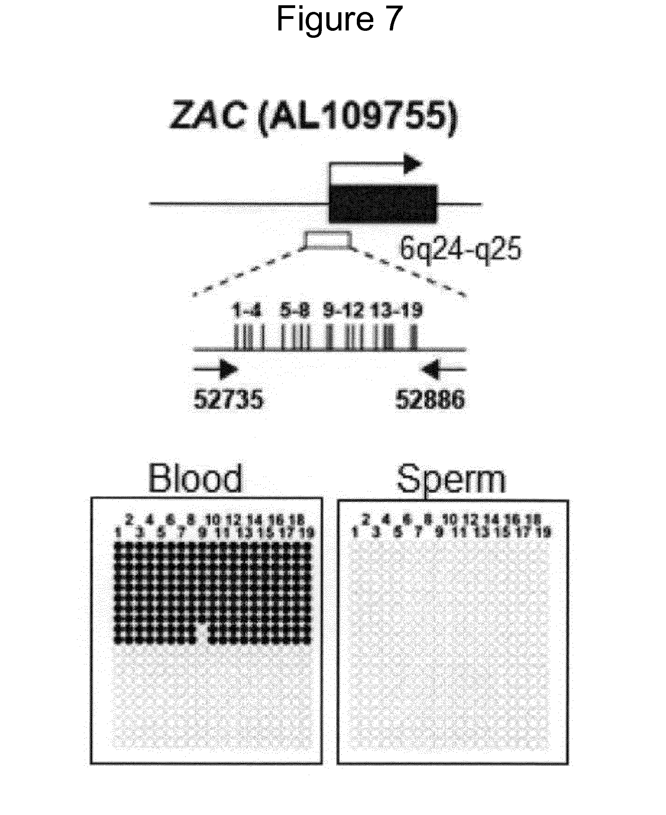

[Figure 7] Figure 7 is a view showing the results obtained by analyzing the methylated state of the differentially methylation region of ZAC and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

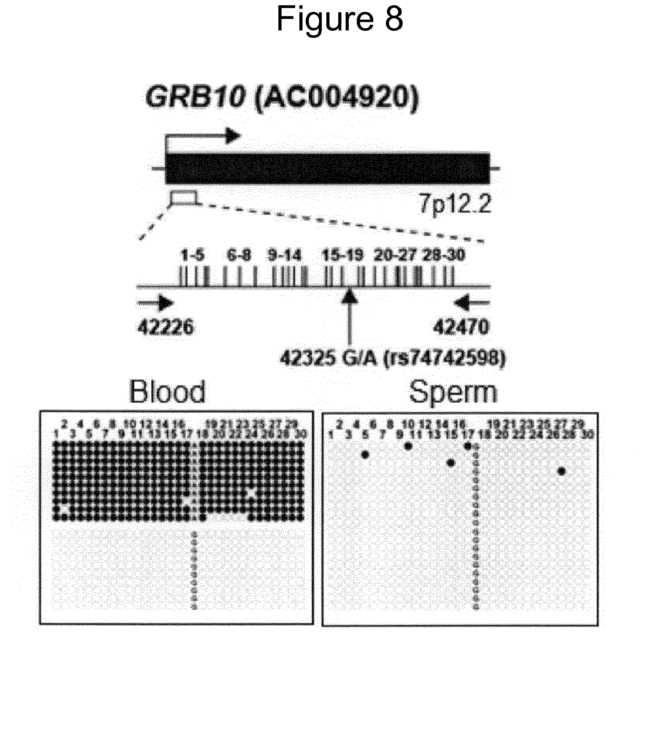

[Figure 8] Figure 8 is a view showing the results obtained by analyzing the methylated state of the differentially methylation region of GRB10 and the methylated state of the differentially methylation region in genomic DNA derived from blood and sperm collected from normal individuals. In the upper figure, the symbol "G/A" indicates a single nucleotide polymorphism site. In addition, in the lower figure, the symbol "○" indicates a non-methylated CpG sequence, and the symbol "●" indicates a methylated CpG sequence.

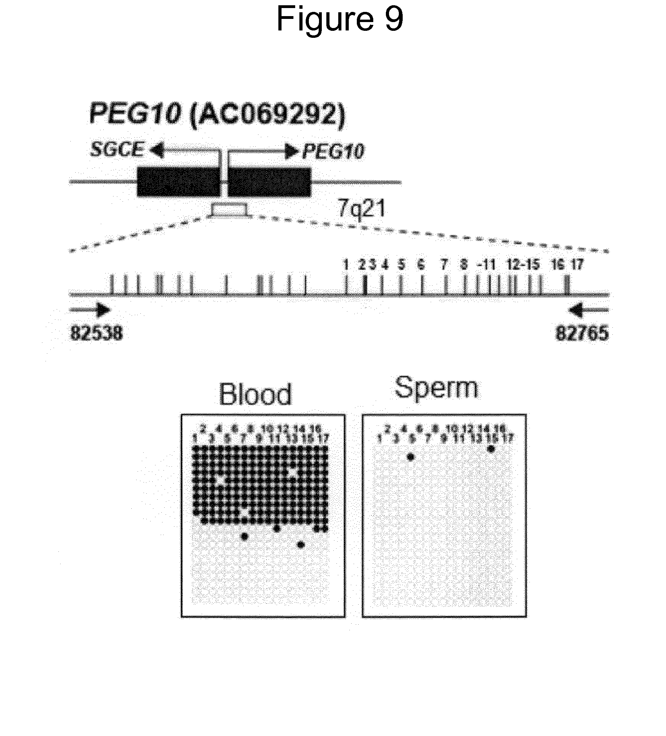

[Figure 9] Figure 9 is a view showing the results obtained by analyzing the methylated state of the differentially methylation region of PEG10 and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

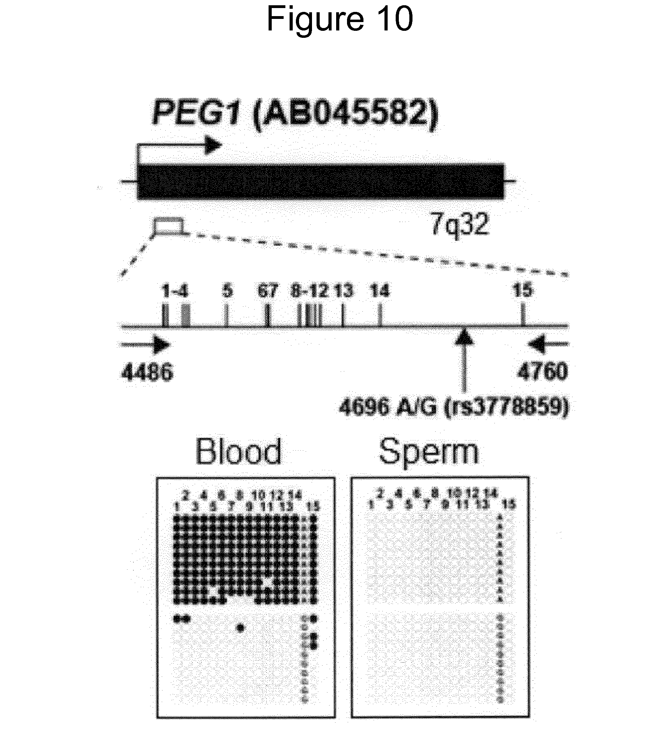

[Figure 10] Figure 10 is a view showing the results obtained by analyzing the methylated state of the differentially methylation region of PEG1 and the methylated state of the differentially methylation region in genomic DNA derived from blood and sperm collected from normal individuals. In the upper figure, the symbol "A/G" indicates a single nucleotide polymorphism site. In addition, in the lower figure, the symbol "○" indicates a non-methylated CpG sequence, and the symbol "●" indicates a methylated CpG sequence.

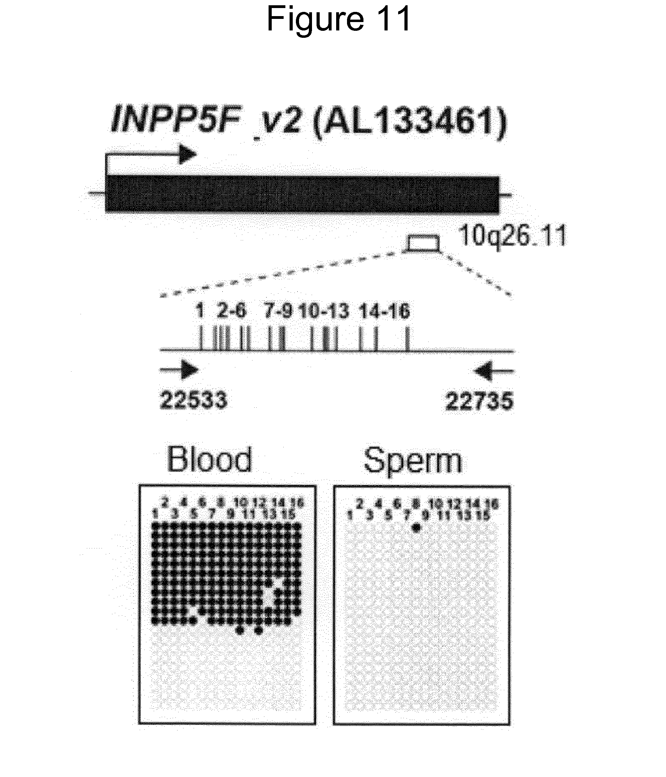

[Figure 11] Figure 11 is a view showing the results obtained by analyzing the methylated state of the differentially methylation region of INPP5Fv2 and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

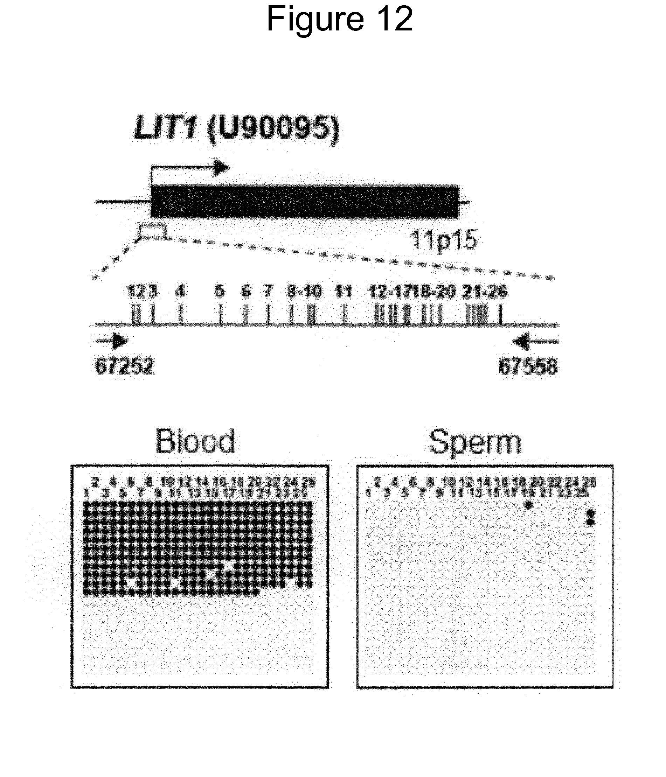

[Figure 12] Figure 12 is a view showing the results obtained by analyzing the methylated state of the differentially methylation region of LIT1 and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

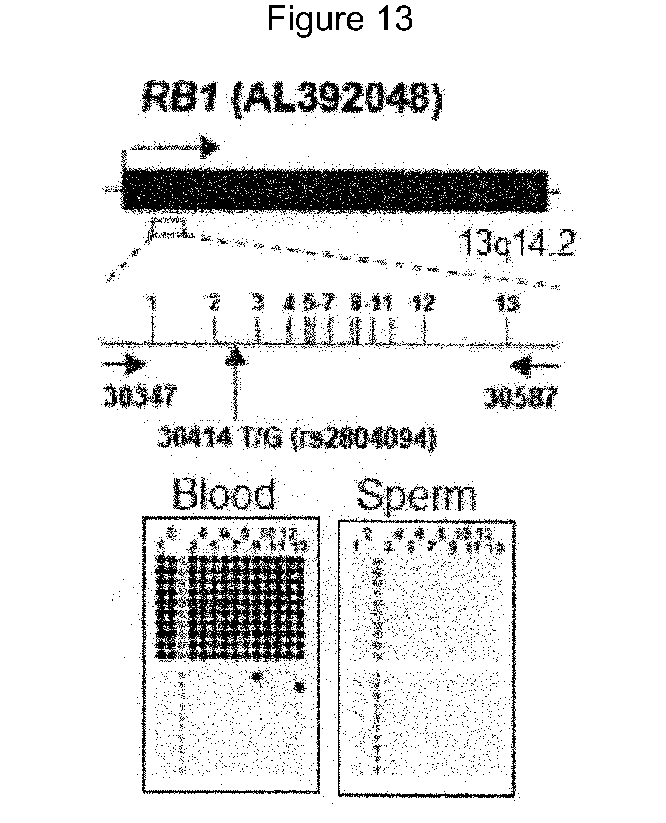

[Figure 13] Figure 13 is a view showing the results obtained by analyzing the methylated state of the differentially methylation region of RB1 and the methylated state of the differentially methylation region in genomic DNA derived from blood and sperm collected from normal individuals. In the upper figure, the symbol "T/G" indicates a single nucleotide polymorphism site. In addition, in the lower figure, the symbol "○" indicates a non-methylated CpG sequence, and the symbol "●" indicates a methylated CpG sequence.

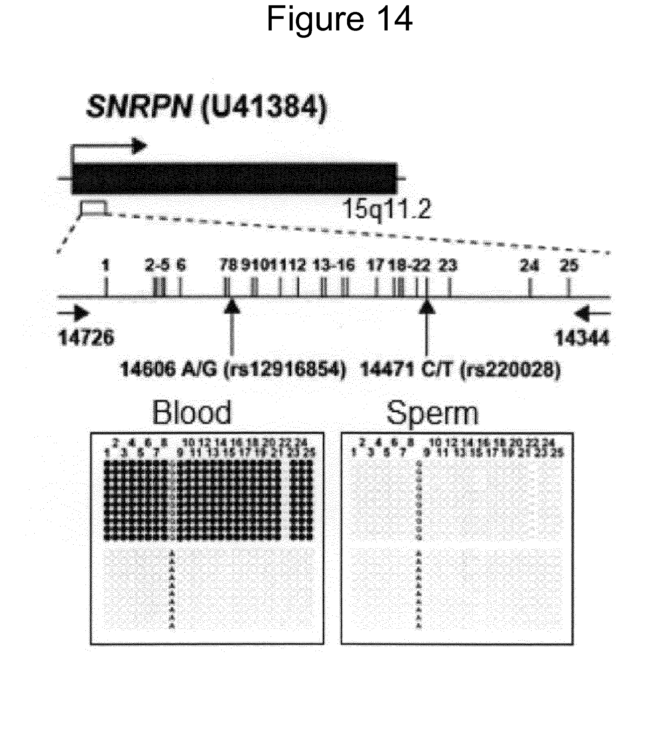

[Figure 14] Figure 14 is a view showing the results obtained by analyzing the methylated state of the differentially methylation region of SNRPN and the methylated state of the differentially methylation region in genomic DNA derived from blood and sperm collected from normal individuals. In the upper figure, the symbols "A/G" and "C/T" each indicate a single nucleotide polymorphism site. In addition, in the lower figure, the symbol "○" indicates a non-methylated CpG sequence, and the symbol "●" indicates a methylated CpG sequence.

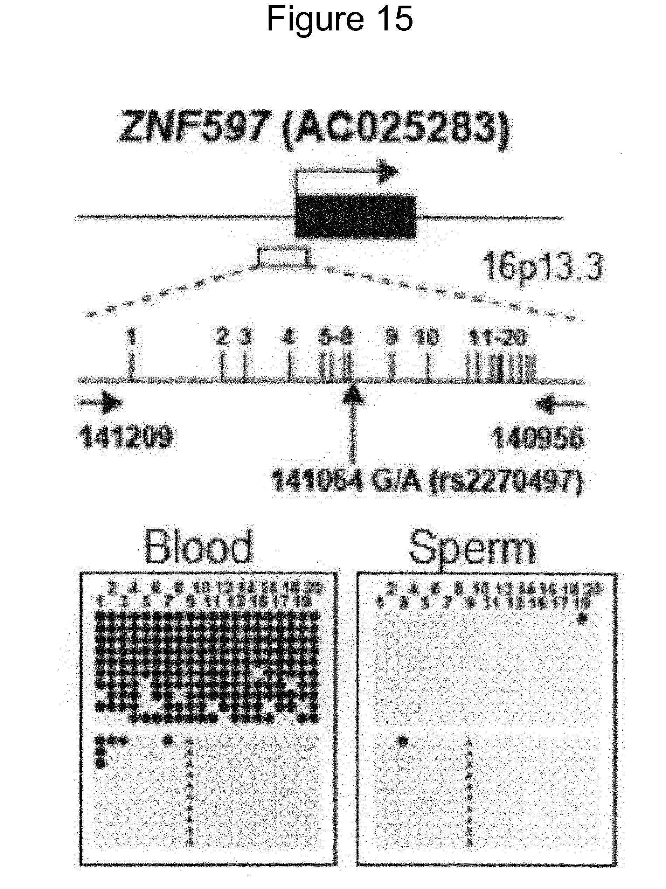

[Figure 15] Figure 15 is a view showing the results obtained by analyzing the methylated state of the differentially methylation region of ZNF597 and the methylated state of the differentially methylation region in genomic DNA derived from blood and sperm collected from normal individuals. In the upper figure, the symbol "G/A" indicates a single nucleotide polymorphism site. In addition, in the lower figure, the symbol "○" indicates a non-methylated CpG sequence, and the symbol "●" indicates a methylated CpG sequence.

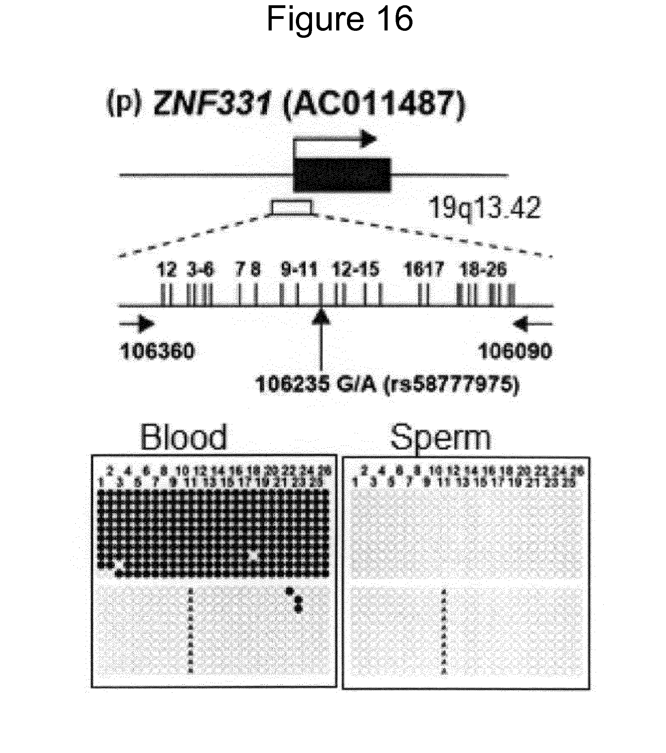

[Figure 16] Figure 16 is a view showing the results obtained by analyzing the methylated state of the differentially methylation region of ZNF331 and the methylated state of the differentially methylation region in genomic DNA derived from blood and sperm collected from normal individuals. In the upper figure, the symbol "A/G" indicates a single nucleotide polymorphism site. Since Figure 16 shows the analysis of a sequence complementary to a DNA sequence corresponding to positions 106360-106090 of GenBank Accession No. AC011487.5, a single nucleotide polymorphism of "C/T" at position 106235 is indicated as "G/A." In addition, in the lower figure, the symbol "○" indicates a non-methylated CpG sequence, and the symbol "●" indicates a methylated CpG sequence.

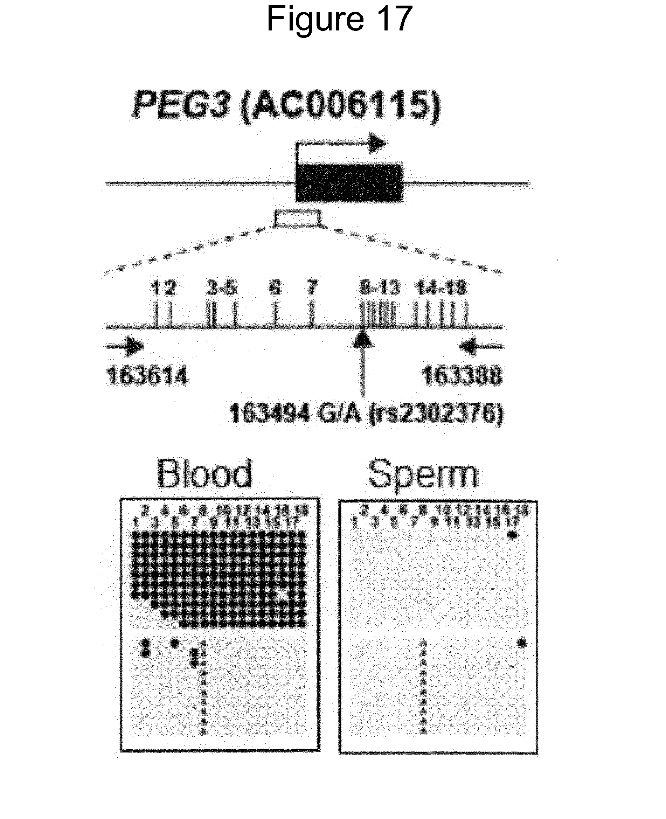

[Figure 17] Figure 17 is a view showing the results obtained by analyzing the methylated state of the differentially methylation region of PEG3 and the methylated state of the differentially methylation region in genomic DNA derived from blood and sperm collected from normal individuals. In the upper figure, the symbol "G/A" indicates a single nucleotide polymorphism site. In addition, in the lower figure, the symbol "○" indicates a non-methylated CpG sequence, and the symbol "●" indicates a methylated CpG sequence.

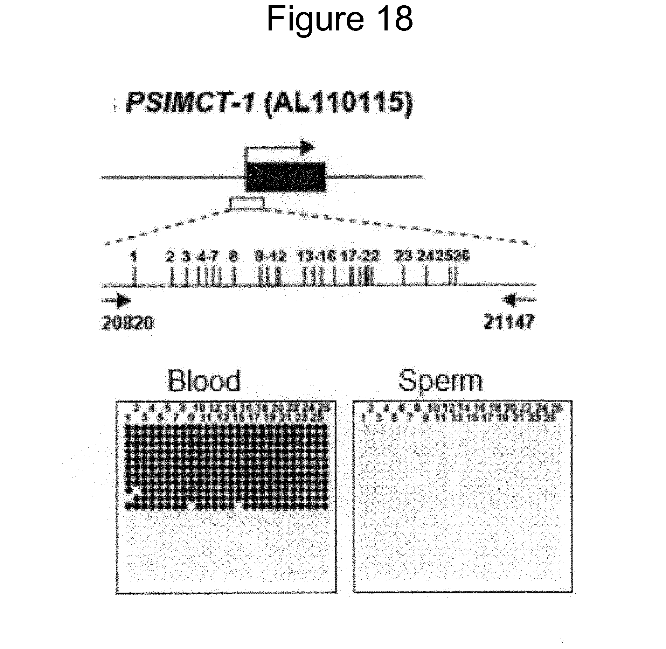

[Figure 18] Figure 18 is a view showing the results obtained by analyzing the methylated state of the differentially methylation region of PSIMCT-1 and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

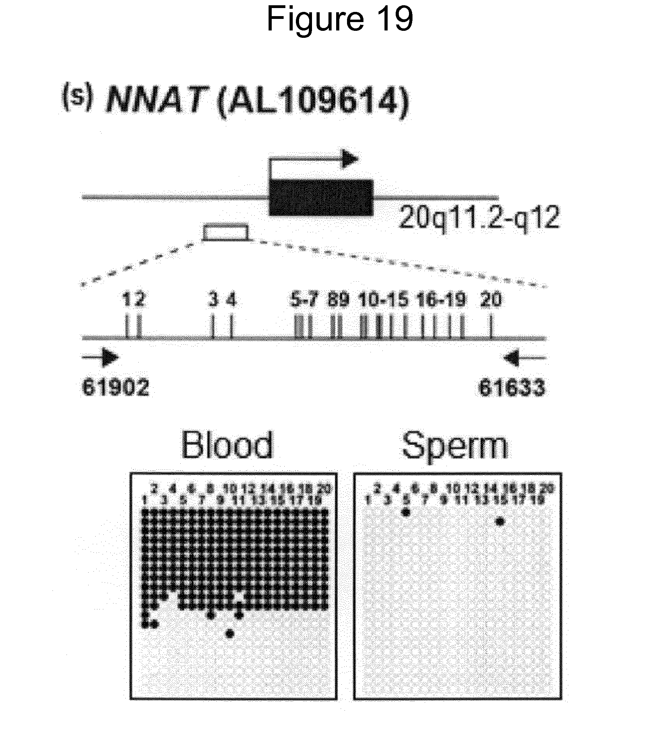

[Figure 19] Figure 19 is a view showing the results obtained by analyzing the methylated state of the differentially methylation region of NNAT and the methylated state of the differentially methylation region in genomic DNA derived from blood and sperm collected from normal individuals. In the upper figure, the symbol "A/G" indicates a single nucleotide polymorphism site. In addition, in the lower figure, the symbol "○" indicates a non-methylated CpG sequence, and the symbol "●" indicates a methylated CpG sequence.

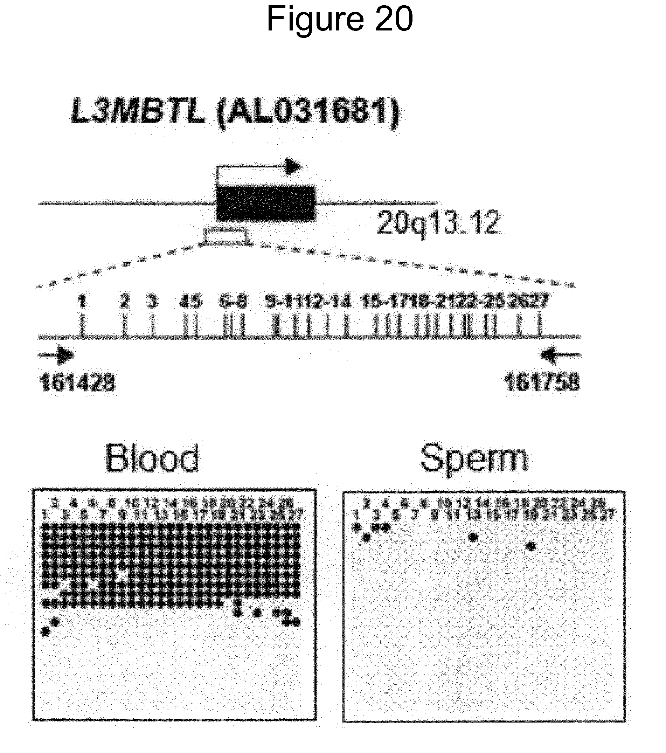

[Figure 20] Figure 20 is a view showing the results obtained by analyzing the methylated state of the differentially methylation region of L3MBTL and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

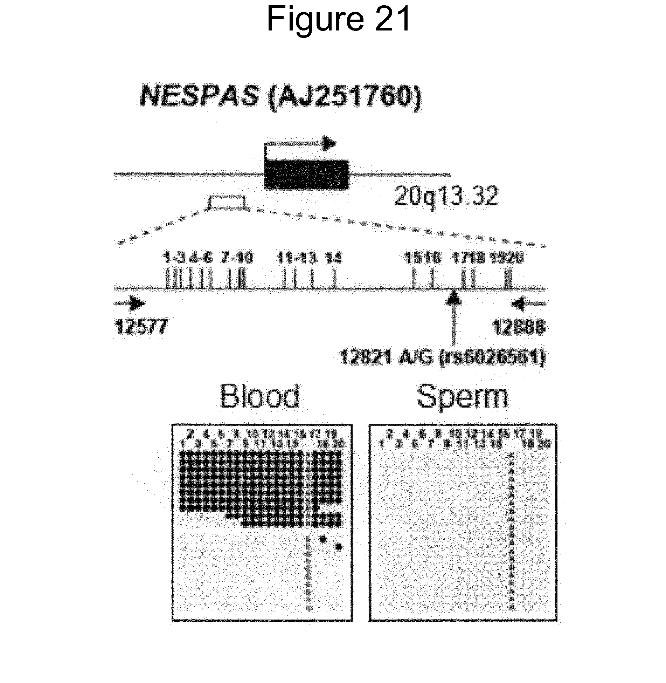

[Figure 21] Figure 21 is a view showing the results obtained by analyzing the methylated state of the differentially methylation region of NESPAS and the methylated state of the differentially methylation region in genomic DNA derived from blood and sperm collected from normal individuals. In the upper figure, the symbol "A/G" indicates a single nucleotide polymorphism site. In addition, in the lower figure, the symbol "○" indicates a non-methylated CpG sequence, and the symbol "●" indicates a methylated CpG sequence.

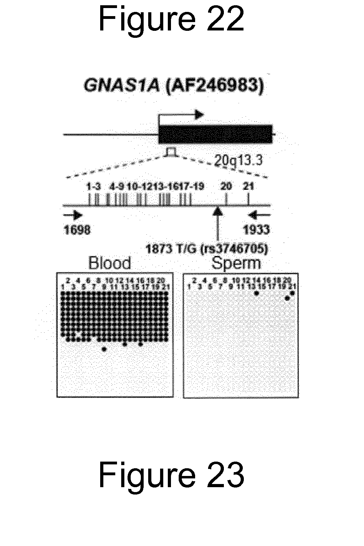

[Figure 22] Figure 22 is a view showing the results obtained by analyzing the methylated state of the differentially methylation region of GNAS1A and the methylated state of the differentially methylation region in genomic DNA derived from blood and sperm collected from normal individuals. In the upper figure, the symbol "T/G" indicates a single nucleotide polymorphism site. In addition, in the lower figure, the symbol "○" indicates a non-methylated CpG sequence, and the symbol "●" indicates a methylated CpG sequence.

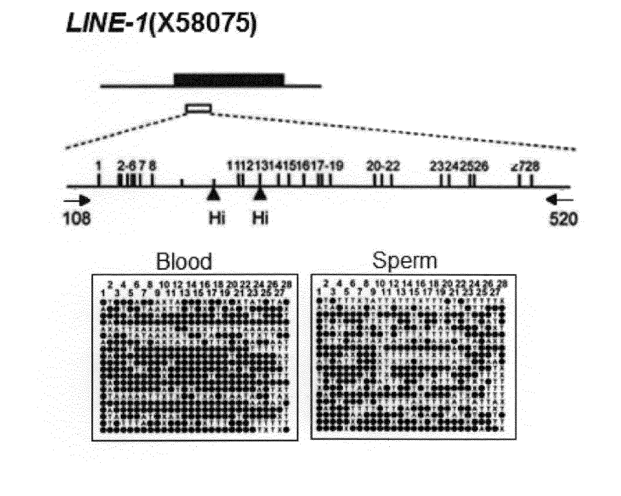

[Figure 23] Figure 23 is a view showing the results obtained by analyzing the methylated state of the differentially methylation region of LINE-1 and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

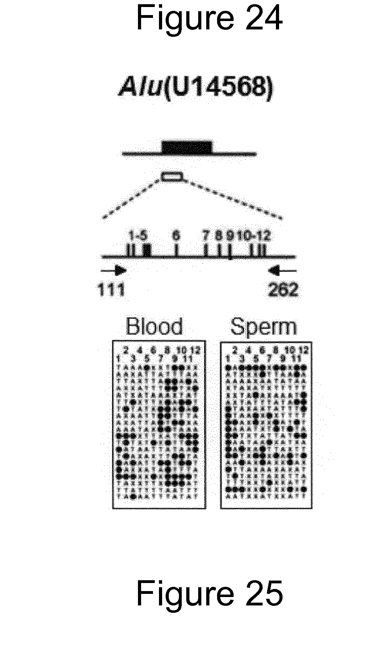

[Figure 24] Figure 24 is a view showing the results obtained by analyzing the methylated state of the differentially methylation region of Alu and the methylated state of the differentially methylation region in genomic DNA derived from blood and sperm collected from normal individuals. In the lower figure, the symbol "○" indicates a non-methylated CpG sequence, and the symbol "●" indicates a methylated CpG sequence.

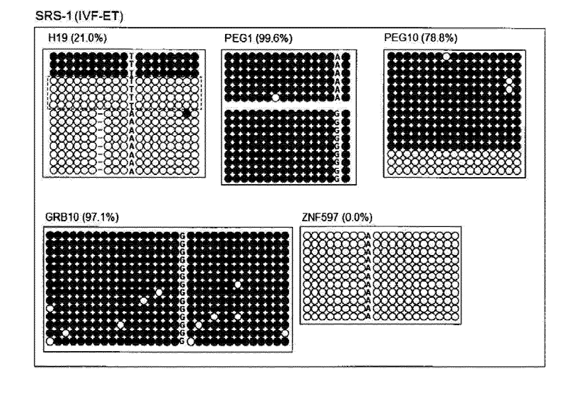

[Figure 25] Figure 25 is a view showing the results obtained by analyzing the methylated state of the differentially methylation region of each of H19, PEG1, PEG10, GRB10, and ZNF597 in genomic DNA derived from blood collected from SRS patients (case 1). In the figure, the symbol "○" indicates a non-methylated CpG sequence, and the symbol "●" indicates a methylated CpG sequence.

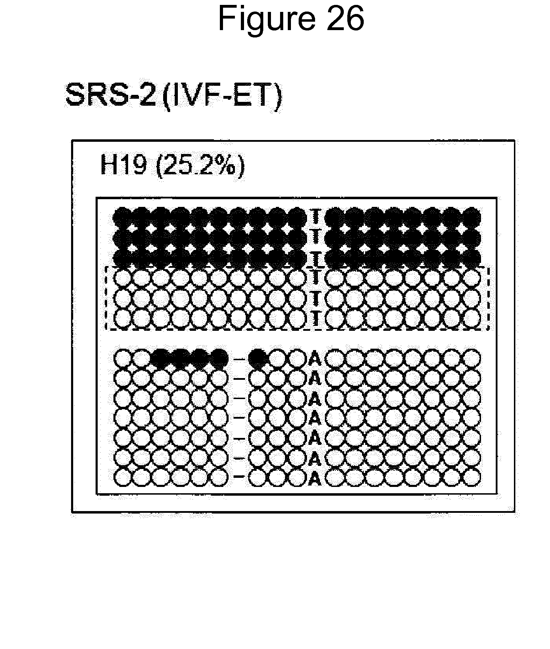

[Figure 26] Figure 26 is a view showing the results obtained by analyzing the methylated state of the differentially methylation region of H19 in genomic DNA derived from blood collected from SRS patients (case 2). In the figure, the symbol "○" indicates a non-methylated CpG sequence, and the symbol "●" indicates a methylated CpG sequence.

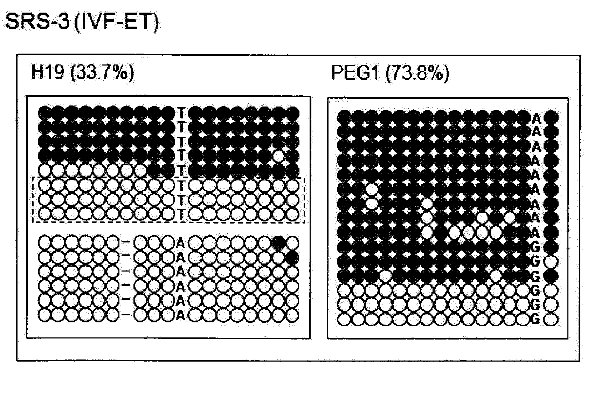

[Figure 27] Figure 27 is a view showing the results obtained by analyzing the methylated state of the differentially methylation region of each of H19 and PEG1 in genomic DNA derived from blood collected from SRS patients (case 3). In the figure, the symbol "○" indicates a non-methylated CpG sequence, and the symbol "●" indicates a methylated CpG sequence.

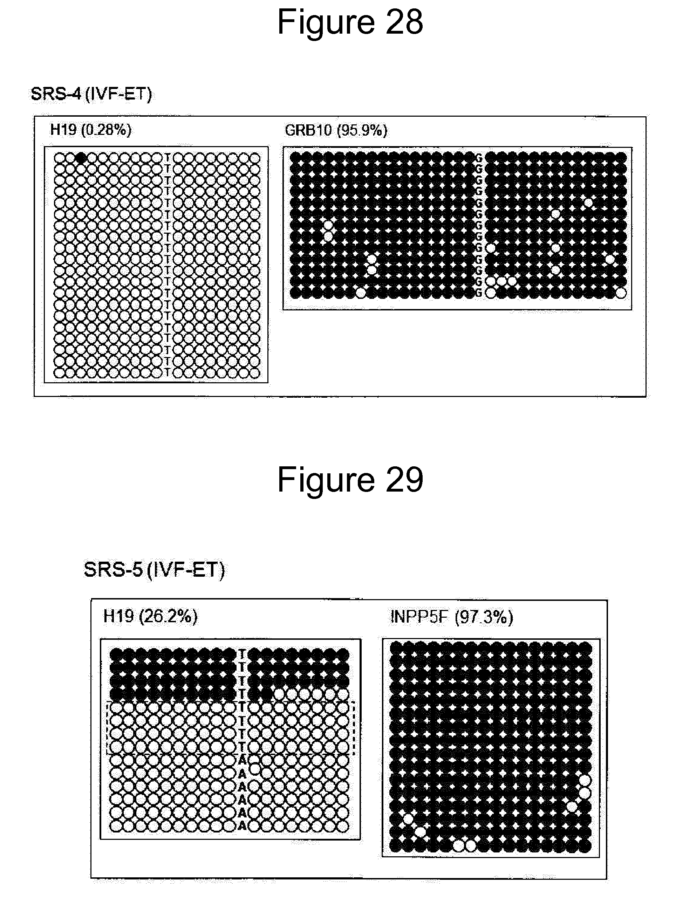

[Figure 28] Figure 28 is a view showing the results obtained by analyzing the methylated state of the differentially methylation region of each of H19 and GRB10 in genomic DNA derived from blood collected from SRS patients (case 4). In the figure, the symbol "○" indicates a non-methylated CpG sequence, and the symbol "●" indicates a methylated CpG sequence.
[Figure 29] Figure 29 is a view showing the results obtained by analyzing the methylated state of the differentially methylation region of each of H19 and INPP5FV2 in genomic DNA derived from blood collected from SRS patients (case 5). In the figure, the symbol "○" indicates a non-methylated CpG sequence, and the symbol "●" indicates a methylated CpG sequence.

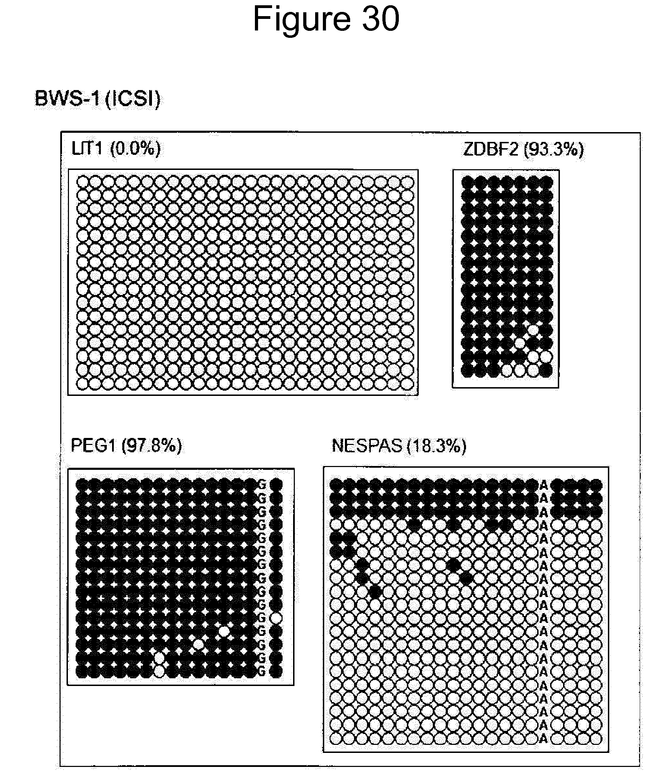

[Figure 30] Figure 30 is a view showing the results obtained by analyzing the methylated state of the differentially methylation region of each of LIT1, ZDBF2, PEG1, and NESPAS in genomic DNA derived from blood collected from BWS patients (case 1). In the figure, the symbol "○" indicates a non-methylated CpG sequence, and the symbol "●" indicates a methylated CpG sequence.

### Mode of Carrying Out the Invention

The method of the present invention for assessing the risk that the progeny of a subject would develop an imprinting disorder (hereinafter also referred to as "the evaluation method of the present invention") is not particularly limited, as long as it is a method comprising the following steps (a) and (b) (however, excluding diagnostic action by a doctor): (a) a step of extracting genomic DNA from sperm collected from the subject; and (b) a step of measuring the methylation level of cytosine residues in a CpG sequence comprised in the differentially methylation region of one or more maternally imprinted genes selected from the group consisting of DIRAS3, NAP1L5, FAM50B, GRB10, INPP5Fv2, RB1, ZNF597, ZNF331, PSIMCT-1, NNAT, L3MBTL, NESPAS, and GNAS1A (wherein hereinafter this gene group may also be referred to as "the maternally imprinted gene group of the present invention"), in the genomic DNA extracted in the step (a). An aspect of the evaluation method of the present invention includes a method of collecting data used for assessing the risk that the progeny of a subject would develop an imprinting disorder. Herein, the term "the progeny of a subject" is used to mean an individual derived from the sperm of a subject, namely, a child of a subject. In addition, the aforementioned "imprinting disorder" is used to mean a disease caused by abnormal methylation in the differentially methylation region of a maternally or paternally imprinted gene, and specifically, preferred examples of the imprinting disorder include Silver-Russell syndrome (SRS), Beckwith-Wiedemann syndrome (BWS), Angelman syndrome (AS), transient neonatal diabetes mellitus (TNDM), and Prader-Willi syndrome (PWS). Among these, Silver-Russell syndrome (SRS) and Beckwith-Wiedemann syndrome (BWS) are particularly preferable.

The above-mentioned step (a) is not particularly limited, as long as it is a step of extracting genomic DNA from sperm collected from a subject. The aforementioned "subject" is not particularly limited, as long as it is a male capable of providing sperm. The subject is preferably a patient with male infertility, and among others, it is particularly preferably a patient with male infertility who is anticipated to receive the treatment of infertility according to ART. Moreover, when the aforementioned "subject" is a patient with male infertility that is azoospermia, sperm is directly collected from testis according to testicular sperm extraction (TESE) or micro dissection testicular sperm extraction (MD-TESE), and it can be then used in the aforementioned step (a).

The above-mentioned step (b) is not particularly limited, as long as it is a step of measuring the methylation level of cytosine residues in a CpG sequence comprised in the differentially methylation region of one or more maternally imprinted genes selected from the maternally imprinted gene group of the present invention, in the genomic DNA extracted in the aforementioned step (a). The phrase "the differentially methylation region of maternally imprinted genes" is used herein to specifically mean, on human genome, a differentially methylation region that is present close to DIRAS3 and is associated with the control of the expression of DIRAS3 (hereinafter also referred to as "the differentially methylation region of DIRAS3"), a differentially methylation region that is present close to NAP1L5 and is associated with the control of the expression of NAP1L5 (hereinafter also referred to as "the differentially methylation region of NAP1L5"), a differentially methylation region that is present close to FAM50B and is associated with the control of the expression of FAM50B (hereinafter also referred to as "the differentially methylation region of FAM50B"), a differentially methylation region that is present close to GRB10 and is associated with the control of the expression of GRB10 (hereinafter also referred to as "the differentially methylation region of GRB10"), a differentially methylation region that is present close to INPP5Fv2 and is associated with the control of the expression of INPP5Fv2 (hereinafter also referred to as "the differentially methylation region of INPP5Fv2"), a differentially methylation region that is present close to RB1 and is associated with the control of the expression of RB1 (hereinafter also referred to as "the differentially methylation region of RB1"), a differentially methylation region that is present close to ZNF597 and is associated with the control of the expression of ZNF597 (hereinafter also referred to as "the differentially methylation region of ZNF597"), a differentially methylation region that is present close to ZNF331 and is associated with the control of the expression of ZNF331 (hereinafter also referred to as "the differentially methylation region of ZNF331"), a differentially methylation region that is present close to PSIMCT-1 and is associated with the control of the expression of PSIMCT-1 (hereinafter also referred to as "the differentially methylation region of PSIMCT-1"), a differentially methylation region that is present close to NNAT and is associated with the control of the expression of NNAT (hereinafter also referred to as "the differentially methylation region of NNAT"), a differentially methylation region that is present close to L3MBTL and is associated with the control of the expression of L3MBTL (hereinafter also referred to as "the differentially methylation region of L3MBTL"), a differentially methylation region that is present close to NESPAS and is associated with the control of the expression of NESPAS (hereinafter also referred to as "the differentially methylation region of NESPAS"), or a differentially methylation region that is present close to GNAS1A and is associated with the control of the expression of GNAS1A (hereinafter also referred to as "the differentially methylation region of GNAS1A").

More specifically, a preferred example of the aforementioned "differentially methylation region of DIRAS3" is a region at positions 1849-2197 (SEQ ID NO: 1) in the nucleotide sequence of human chromosome 1 registered under GenBank Accession No. AF202543.1 (date of update: August 13, 2001). In addition, preferred examples of the CpG sequence comprised in the aforementioned "differentially methylation region of DIRAS3" include CpG sequences at positions 1873-1874, 1893-1894, 1900-1901, 1902-1903, 1905-1906, 1912-1913, 1947-1948, 1954-1955, 1960-1961, 1991-1992, 1997-1998, 2004-2005, 2014-2015, 2021-2022, 2034-2035, 2036-2037, 2072-2073, 2074-2075, 2086-2087, 2093-2094, 2120-2121, 2128-2129, and 2150-2151, in the nucleotide sequence of human chromosome 1 registered under GenBank Accession No. AF202543.1 (date of update: August 13, 2001).

A preferred example of the aforementioned "differentially methylation region of NAP1L5" is a region at positions 35225-35572 (SEQ ID NO: 2) in the nucleotide sequence of human chromosome 4 that was registered under GenBank Accession No. AC108065.3 (date of update: May 24, 2002). In addition, preferred examples of the CpG sequence comprised in the aforementioned "differentially methylation region of NAP1L5" include CPG sequences at positions 35258-35259, 35260-35261, 35279-35280, 35281-35282, 35303-35304, 35308-35309, 35315-35316, 35333-35334, 35342-35343, 35345-35346, 35354-35355, 35369-35370, 35387-35388, 35392-35393, 35413-35414, 35435-35436, 35452-35453, 35475-35476, 35481-35482, 35491-35492, 35497-35498, 35501-35502, 35505-35506, 35507-35508, 35516-35517, 35523-35524, 35525-35526, 35531-35532, 35544-35545, and 35546-35547, in the nucleotide sequence of human chromosome 4 registered under GenBank Accession No. AC108065.3 (date of update: May 24, 2002).

Moreover, when the thymine residue at position 35330 in the nucleotide sequence of human chromosome 4 registered under GenBank Accession No. AC108065.3 (date of update: May 24, 2002) is mutated to a cytosine residue, preferred examples of the CpG sequence comprised in the aforementioned "differentially methylation region of NAP1L5" include CpG sequences at positions 35258-35259, 35260-35261, 35279-35280, 35281-35282, 35303-35304, 35308-35309, 35315-35316, 35329-35330, 35333-35334, 35342-35343, 35345-35346, 35354-35355, 35369-35370, 35387-35388, 35392-35393, 35413-35414, 35435-35436, 35452-35453, 35475-35476, 35481-35482, 35491-35492, 35497-35498, 35501-35502, 35505-35506, 35507-35508, 35516-35517, 35523-35524, 35525-35526, 35531-35532, 35544-35545, and 35546-35547, in the nucleotide sequence of human chromosome 4 registered under GenBank Accession No. AC108065.3 (date of update: May 24, 2002).

A preferred example of the aforementioned "differentially methylation region of FAM50B" is a region at positions 350-683 (SEQ ID NO: 3) in the nucleotide sequence of human chromosome 6 that was registered under GenBank Accession No. Y18504.1 (date of update: November 14, 2006). In addition, preferred examples of the CpG sequence comprised in the aforementioned "differentially methylation region of FAM50B" include CpG sequences at positions 379-380, 384-385, 401-402, 419-420, 434-435, 438-439, 457-458, 471-472, 488-489, 498-499, 518-519, 539-540, 541-542, 549-550, 565-566, 582-583, 601-602, 609-610, 619-620, 643-644, and 649-650, in the nucleotide sequence of human chromosome 6 registered under GenBank Accession No. Y18504.1 (date of update: November 14, 2006). In addition,

A preferred example of the aforementioned "differentially methylation region of GRB10" is a region at positions 42226-42470 (SEQ ID NO: 4) in the nucleotide sequence of human chromosome 7 that was registered under GenBank Accession No. AC004920.2 (date of update: October 15, 2003). In addition, preferred examples of the CpG sequence comprised in the aforementioned "differentially methylation region of GRB10" include CpG sequences at positions 42251-42252, 42256-42257, 42264-42265, 42273-42274, 42275-42276, 42277-42278, 42284-42285, 42288-42289, 42290-42291, 42298-42299, 42305-42306, 42313-42314, 42316-42317, 42328-42329, 42335-42336, 42339-42340, 42353-42354, 42355-42356, 42361-42362, 42365-42366, 42369-42370, 42375-42376, 42388-42389, 42399-42400, 42409-42410, 42421-42422, 42423-42424, 42429-42430, 42436-42437, and 42440-42441, in the nucleotide sequence of human chromosome 7 registered under GenBank Accession No. AC004920.2 (date of update: October 15, 2003).

A preferred example of the aforementioned "differentially methylation region of INPP5Fv2" is a region at positions 22533-22735 (SEQ ID NO: 5) in the nucleotide sequence of human chromosome 10 that was registered under GenBank Accession No. AL133461.10 (date of update: January 13, 2009). In addition, preferred examples of the CpG sequence comprised in the aforementioned "differentially methylation region of INPP5Fv2" include CpG sequences at positions 22556-22557, 22564-22565, 22567-22568, 22571-22572, 22579-22580, 22583-22584, 22595-22596, 22601-22602, 22603-22604, 22619-22620, 22626-22627, 22628-22629, 22633-22634, 22647-22648, 22656-22657, and 22674-22675, in the nucleotide sequence of human chromosome 10 registered under GenBank Accession No. AL133461.10 (date of update: January 13, 2009).

A preferred example of the aforementioned "differentially methylation region of RB1" is a region at positions 30347-30587 (SEQ ID NO: 6) in the nucleotide sequence of human chromosome 13 that was registered under GenBank Accession No. AL392048.9 (date of update: January 13, 2009). In addition, preferred examples of the CpG sequence comprised in the aforementioned "differentially methylation region of RB1" include CpG sequences at positions 30370-30371, 30403-30404, 30426-30427, 30443-30444, 30452-30453, 30455-30456, 30464-30465, 30476-30477, 30479-30480, 30487-30488, 30497-30498, 30514-30515, and 30558-30559, in the nucleotide sequence of human chromosome 13 registered under GenBank Accession No. AL392048.9 (date of update: January 13, 2009).

A preferred example of the aforementioned "differentially methylation region of ZNF597" is a region at positions 140956-141209 (SEQ ID NO: 7) in the nucleotide sequence of human chromosome 16 that was registered under GenBank Accession No. AC025283.6 (date of update: September 5, 2002). In addition, preferred examples of the CpG sequence comprised in the aforementioned "differentially methylation region of ZNF597" include CpG sequences at positions 140981-140982, 140984-140985, 140988-140989, 140992-140993, 140997-140998, 140999-141000, 141002-141003, 141009-141010, 141014-141015, 141034-141035, 141052-141053, 141063-141064, 141073-141074, 141076-141077, 141082-141083, 141087-141088, 141103-141104, 141126-141127, 141137-141138, and 141183-141184, in the nucleotide sequence of human chromosome 16 registered under GenBank Accession No. AC025283.6 (date of update: September 5, 2002).

Moreover, when the guanine residue at position 141064 in the nucleotide sequence of human chromosome 16 registered under GenBank Accession No. AC025283.6 (date of update: September 5, 2002) is mutated to a adenine residue, preferred examples of the CpG sequence comprised in the aforementioned "differentially methylation region of ZNF597" include positions 140981-140982, 140984-140985, 140988-140989, 140992-140993, 140997-140998, 140999-141000, 141002-141003, 141009-141010, 141014-141015, 141034-141035, 141052-141053, 141073-141074, 141076-141077, 141082-141083, 141087-141088, 141103-141104, 141126-141127, 141137-141138, and 141183-141184, in the nucleotide sequence of human chromosome 16 registered under GenBank Accession No. AC025283.6 (date of update: September 5, 2002).

A preferred example of the aforementioned "differentially methylation region of ZNF331" is a region at positions 106090-106360 (SEQ ID NO: 8) in the nucleotide sequence of human chromosome 19 that was registered under GenBank Accession No. AC011487.5 (date of update: February 28, 2001). In addition, preferred examples of the CpG sequence comprised in the aforementioned "differentially methylation region of ZNF331" include CpG sequences at positions 106115-106116, 106117-106118, 106123-106124, 106127-106128, 106129-106130, 106138-106139, 106142-106143, 106147-106148, 106149-106150, 106168-106169, 106173-106174, 106197-106198, 106207-106208, 106220-106221, 106225-106226, 106235-106236, 106249-106250, 106259-106260, 106275-106276, 106285-106286, 106303-106304, 106307-106308, 106313-106314, 106317-106318, 106328-106329, and 106333-106334, in the nucleotide sequence of human chromosome 19 registered under GenBank Accession No. AC011487.5 (date of update: February 28, 2001).

Moreover, when the cytosine residue at position 106235 in the nucleotide sequence of human chromosome 19 registered under GenBank Accession No. AC011487.5 (date of update: February 28, 2001) is mutated to a thymine residue, preferred examples of the CpG sequence comprised in the aforementioned "differentially methylation region of ZNF331" include positions 106115-106116, 106117-106118, 106123-106124, 106127-106128, 106129-106130, 106138-106139, 106142-106143, 106147-106148, 106149-106150, 106168-106169, 106173-106174, 106197-106198, 106207-106208, 106220-106221, 106225-106226, 106249-106250, 106259-106260, 106275-106276, 106285-106286, 106303-106304, 106307-106308, 106313-106314, 106317-106318, 106328-106329, and 106333-106334, in the nucleotide sequence of human chromosome 19 registered under GenBank Accession No. AC011487.5 (date of update: February 28, 2001).

A preferred example of the aforementioned "differentially methylation region of PSIMCT-1" is a region at positions 20820-21147 (SEQ ID NO: 9) in the nucleotide sequence of human chromosome 20 that was registered under GenBank Accession No. AL110115.38 (date of update: January 13, 2009). In addition, preferred examples of the CpG sequence comprised in the aforementioned "differentially methylation region of PSIMCT-1" include CpG sequences at positions 20844-20845, 20872-20873, 20883-20884, 20892-20893, 20898-20899, 20903-20904, 20908-20909, 20919-20920, 20939-20940, 20944-20945, 20951-20952, 20953-20954, 20972-20973, 20979-20980, 20985-20986, 20995-20996, 21007-21008, 21009-21010, 21014-21015, 21018-21019, 21020-21021, 21023-21024, 21047-21048, 21064-21065, 21082-21083, and 21086-21087, in the nucleotide sequence of human chromosome 20 registered under GenBank Accession No. AL110115.38 (date of update: January 13, 2009).

A preferred example of the aforementioned "differentially methylation region of NNAT" is a region at positions 61633-61902 (SEQ ID NO: 10) in the nucleotide sequence of human chromosome 20 that was registered under GenBank Accession No. AL109614.28 (date of update: January 13, 2009). In addition, preferred examples of the CpG sequence comprised in the aforementioned "differentially methylation region of NNAT" include CpG sequences at positions 61659-61660, 61666-61667, 61708-61709, 61719-61720, 61757-61758, 61759-61760, 61765-61766, 61778-61779, 61782-61783, 61795-61796, 61797-61798, 61804-61805, 61806-61807, 61812-61813, 61820-61821, 61830-61831, 61837-61838, 61846-61847, 61853-61854, and 61870-61871, in the nucleotide sequence of human chromosome 20 registered under GenBank Accession No. AL109614.28 (date of update: January 13, 2009).

A preferred example of the aforementioned "differentially methylation region of L3MBTL" is a region at positions 161428-161758 (SEQ ID NO: 11) in the nucleotide sequence of human chromosome 20 that was registered under GenBank Accession No. AL031681.16 (date of update: January 13, 2009). In addition, preferred examples of the CpG sequence comprised in the aforementioned "differentially methylation region of L3MBTL" include CpG sequences at positions 161454-161455, 161480-161481, 161497-161498, 161517-161518, 161523-161524, 161541-161542, 161545-161546, 161552-161553, 161571-161572, 161573-161574, 161584-161585, 161592-161593, 161603-161604, 161615-161616, 161633-161634, 161640-161641, 161647-161648, 161658-161659, 161664-161665, 161670-161671, 161679-161680, 161687-161688, 161690-161691, 161700-161701, 161705-161706, 161720-161721, and 161733-161734, in the nucleotide sequence of human chromosome 20 registered under GenBank Accession No. AL031681.16 (date of update: January 13, 2009).

A preferred example of the aforementioned "differentially methylation region of NESPAS" is a region at positions 12577-12888 (SEQ ID NO: 12) in the nucleotide sequence of human chromosome 20 that was registered under GenBank Accession No. AJ251760.1 (date of update: November 14, 2006). In addition, preferred examples of the CpG sequence comprised in the aforementioned "differentially methylation region of NESPAS" include CpG sequences at positions 12615-12616, 12620-12621, 12624-12625, 12631-12632, 12639-12640, 12646-12647, 12659-12660, 12666-12667, 12668-12669, 12670-12671, 12699-12700, 12706-12707, 12719-12720, 12735-12736, 12792-12793, 12806-12807, 12828-12829, 12835-12836, 12859-12860, and 12862-12863, in the nucleotide sequence of human chromosome 20 registered under GenBank Accession No. AJ251760.1 (date of update: November 14, 2006).

A preferred example of the aforementioned "differentially methylation region of GNAS1A" is a region at positions 1698-1933 (SEQ ID NO: 13) in the nucleotide sequence of human chromosome 20 that was registered under GenBank Accession No. AF246983.1 (date of update: November 20, 2000). In addition, preferred examples of the CpG sequence comprised in the aforementioned "differentially methylation region of GNAS1A" include CpG sequences at positions 1727-1728, 1734-1735, 1737-1738, 1747-1748, 1749-1750, 1757-1758, 1762-1763, 1766-1767, 1769-1770, 1782-1783, 1785-1786, 1791-1792, 1807-1808, 1811-1812, 1815-1816, 1818-1819, 1831-1832, 1836-1837, 1842-1843, 1883-1884, and 1908-1909, in the nucleotide sequence of human chromosome 20 registered under GenBank Accession No. AF246983.1 (date of update: November 20, 2000).

As described above, the aforementioned step (b) is not particularly limited, as long as it is a step of measuring the methylation level of cytosine residues in a CpG sequence comprised in the differentially methylation region of one or more maternally imprinted genes selected from the maternally imprinted gene group of the present invention, in the genomic DNA extracted in the aforementioned step (a). The step (b) is preferably a step of selecting two or more maternally imprinted genes from the maternally imprinted gene group of the present invention and then measuring the methylation level of cytosine residues in a CpG sequence comprised in each of the differentially methylation regions, and is more preferably a step of selecting all (13) maternally imprinted genes from the maternally imprinted gene group of the present invention and then measuring the methylation level of cytosine residues in a CpG sequence comprised in each of the differentially methylation regions. Furthermore, in the aforementioned step (b), the methylation of cytosine residues in at least one CpG sequence from among CpG sequences comprised in the differentially methylation region of the selected maternally imprinted gene(s) may be measured. However, it is more preferable to measure the methylation of cytosine residues in a plurality of CpG sequences, and it is even more preferable to measure the methylation of cytosine residues in all CpG sequences comprised in the differentially methylation region of the selected maternally imprinted gene(s). Further, the aforementioned step (b) of the present invention may include a step of measuring the methylation level of cytosine residues in a CpG sequence(s) comprised in the differentially methylation region of other known paternally or maternally imprinted genes, as well as the differentially methylation region of the maternally imprinted gene group of the present invention. Specifically, the step (b) may include a step of measuring the methylation level of cytosine residues in a CpG sequence(s) comprised in the differentially methylation region of paternally imprinted genes such as H19 and ZDBF2, or maternally imprinted genes such as LIT1.

As described in Examples below, it has been made clear that, in the case of the differentially methylation region of the aforementioned "maternally imprinted gene group of the present invention," almost 100% methylated alleles and almost 100% demethylated alleles are present at a ratio of 1:1 in genomic DNA derived from blood collected from normal individuals, but that in genomic DNA derived from sperm collected from normal individuals, highly methylated alleles are not present, and only almost 100% demethylated alleles are present. From these results, with regard to the aforementioned "maternally imprinted gene group of the present invention," it is considered that only the paternal allele genes function and the maternal allele genes do not function in normal individuals. As such, if the differentially methylation region of the aforementioned "maternally imprinted gene group of the present invention" is highly methylated in genomic DNA from sperm, it is considered that there is a risk that a child derived from the aforementioned sperm would develop an imprinting disorder because the paternal allele genes, which should originally function, cannot function.

Accordingly, in the evaluation method of the present invention, if the measurement results that the methylation level of cytosine residues in a CpG sequence comprised in one or more differentially methylation regions selected from the maternally imprinted gene group of the present invention is high in DNA genome derived from the sperm of a subject are obtained in the step (b), it can be considered that there is a risk that the progeny of the subject would develop an imprinting disorder, or that the risk is high. On the other hand, if the measurement results that the methylation level of cytosine residues in a CpG sequence comprised in one or more differentially methylation regions selected from the maternally imprinted gene group of the present invention is low in DNA genome derived from the sperm of a subject are obtained in the step (b), it can be considered that there is a low risk that the progeny of the subject would develop an imprinting disorder. The term "methylation level" is used herein to mean the ratio (methylation rate) of the number of methylated CpG sequences to the total number of CpG sequences comprised in the differentially methylation region as a measurement target in the aforementioned step (b). That is, the phrase "the methylation level is high" means that the methylation rate is, for example, 50% or more, more preferably 70% or more, and even more preferably 90% or more. In contrast, the phrase "the methylation level is low" means that the methylation rate is, for example, 20% or less, more preferably 10% or less, and even more preferably 5% or less.

In the aforementioned step (b), the method of measuring the methylation level is not particularly limited. Specifically, preferred examples of the method of measuring the methylation level include a bisulfite sequence method, a CGH (Comparative Genomic Hybridization) method, a MassArray method, a Methylation-specific PCR method, a MethylLight method, a Southern blot method, a COBRA (Combined Bisulfite Restriction Analysis) method, a BAMCA (Bacterial artificial chromosome array-based methylated CpG island amplification) method, and a methylation chip method. Among these methods, the bisulfite sequence method is particularly preferable. The primer set used to the aforementioned bisulfite sequence method is not particularly limited, as long as it is a primer set designed to be able to specifically amplify a region comprising at least one CpG sequence comprised in the differentially methylation region of the maternally imprinted gene of the present invention. Specifically, preferred examples of such a primer set include: i) a primer set for amplifying the differentially methylation region of DIRAS3, which consists of the nucleotide sequences shown in SEQ ID NOS: 14 and 15; ii) a primer set for amplifying the differentially methylation region of NAP1L5, which consists of the nucleotide sequences shown in SEQ ID NOS: 16 and 17; iii) a primer set for amplifying the differentially methylation region of FAM50B, which consists of the nucleotide sequences shown in SEQ ID NOS: 18 and 19; iv) a primer set for amplifying the differentially methylation region of GRB10, which consists of the nucleotide sequences shown in SEQ ID NOS: 20 and 21; v) a primer set for amplifying the differentially methylation region of INPP5Fv2, which consists of the nucleotide sequences shown in SEQ ID NOS: 22 and 23; vi) a primer set for amplifying the differentially methylation region of RB1, which consists of the nucleotide sequences shown in SEQ ID NOS: 24 and 25; vii) a primer set for amplifying the differentially methylation region of ZNF597, which consists of the nucleotide sequences shown in SEQ ID NOS: 26 and 27; viii) a primer set for amplifying the differentially methylation region of ZNF331, which consists of the nucleotide sequences shown in SEQ ID NOS: 28 and 29; ix) a primer set for amplifying the differentially methylation region of PSIMCT-1, which consists of the nucleotide sequences shown in SEQ ID NOS: 30 and 31; x) a primer set for amplifying the differentially methylation region of NNAT, which consists of the nucleotide sequences shown in SEQ ID NOS: 32 and 33; xi) a primer set for amplifying the differentially methylation region of L3MBTL, which consists of the nucleotide sequences shown in SEQ ID NOS: 34 and 35; xii) a primer set for amplifying the differentially methylation region of NESPAS, which consists of the nucleotide sequences shown in SEQ ID NOS: 36 and 37; and xiii) a primer set for amplifying the differentially methylation region of GNAS1A, which consists of the nucleotide sequences shown in SEQ ID NOS: 38 and 39.

Moreover, the test kit of the present invention for assessing the risk that the progeny of a subject would develop an imprinting disorder (hereinafter also referred to as "the test kit of the present invention") is not particularly limited, as long as it comprises one or more primer sets selected from the following i) to xiii) : i) a primer set for amplifying the differentially methylation region of DIRAS3, which consists of the nucleotide sequences shown in SEQ ID NOS: 14 and 15; ii) a primer set for amplifying the differentially methylation region of NAP1L5, which consists of the nucleotide sequences shown in SEQ ID NOS: 16 and 17; iii) a primer set for amplifying the differentially methylation region of FAM50B, which consists of the nucleotide sequences shown in SEQ ID NOS: 18 and 19; iv) a primer set for amplifying the differentially methylation region of GRB10, which consists of the nucleotide sequences shown in SEQ ID NOS: 20 and 21; v) a primer set for amplifying the differentially methylation region of INPP5Fv2, which consists of the nucleotide sequences shown in SEQ ID NOS: 22 and 23; vi) a primer set for amplifying the differentially methylation region of RB1, which consists of the nucleotide sequences shown in SEQ ID NOS: 24 and 25; vii) a primer set for amplifying the differentially methylation region of ZNF597, which consists of the nucleotide sequences shown in SEQ ID NOS: 26 and 27; viii) a primer set for amplifying the differentially methylation region of ZNF331, which consists of the nucleotide sequences shown in SEQ ID NOS: 28 and 29; ix) a primer set for amplifying the differentially methylation region of PSIMCT-1, which consists of the nucleotide sequences shown in SEQ ID NOS: 30 and 31; x) a primer set for amplifying the differentially methylation region of NNAT, which consists of the nucleotide sequences shown in SEQ ID NOS: 32 and 33; xi) a primer set for amplifying the differentially methylation region of L3MBTL, which consists of the nucleotide sequences shown in SEQ ID NOS: 34 and 35; xii) a primer set for amplifying the differentially methylation region of NESPAS, which consists of the nucleotide sequences shown in SEQ ID NOS: 36 and 37; and xiii) a primer set for amplifying the differentially methylation region of GNAS1A, which consists of the nucleotide sequences shown in SEQ ID NOS: 38 and 39. Furthermore, the test kit of the present invention may also comprise a reagent for carrying out the bisulfite sequence method and the like.

Hereinafter, the present invention will be more specifically described in the following Examples. However, these Examples are not intended to limit the technical scope of the present invention.

### Examples

### Example 1

Blood and sperm were collected from normal individuals and patients with imprinting disorder, and genomic DNA was then extracted according to a standard method described in Kobayashi et al., (Hum Mol Genet. 2007; 16: 2542-51), etc. It is to be noted that the present study was carried out with the consent of the patients under approval of the Ethics Committee.

### Example 2

### [Analysis using sperm and blood derived from normal individuals]

Using sperm and blood samples derived from 20 normal individuals, single nucleotide polymorphism (SNP) was analyzed with regard to the previously reported 22 gDMRs (human germline Differentially Methylated Regions), namely, ZDBF2, H19, GTL2, ZAC, PEG1, LIT1, SNRPN, LINE-1, Alu, DIRAS3, NAP1L5, FAM50B, GRB10, INPP5Fv2, RB1, ZNF597, ZNF331, PSIMCT-1, NNAT, L3MBTL, NESPAS, and GNAS1A. In addition, the analysis was also carried out regarding repeated sequences (LINEI and Alu). Moreover, the methylated state of each of the above described 22 gDMRs in genomic DNA derived from the sperm and blood samples was confirmed according to a bisulfite sequence method. The names of the analyzed genes or regions and primer sequences used in the SNP analysis and the bisulfite sequence method are shown in Table 1.

**[Table 1]**

| **Gene** | **Target** | **Primer sequence (5'-3')** | | **Ampl (bp)** | **Anneal ing** | **Accession No.** | **chromosomal location** |
|---|---|---|---|---|---|---|---|
| ZDBF2 | BS | ZDBF2 BSF1 | GTTTTGTTAGTTAGATTGGAAAATA (SEQ ID NO.40) | 210 | 57 | AC007383 | 2q33.3 |
| | | ZDBF2 BSR1 | AAAATAATAATTACCTAAAAATAAAAAC (SEQ ID NO.41) | | | | |
| H19 | BS | H19 F2 | TATATGGGTATTTTTGGAGGTTTTT (SEQ ID NO.42) | 220 | 57 | AF125183 | 11p15.5 |
| | | H19 R1 | ATAAATATCCTATTCCCAAATAACCCC (SEQ ID NO.43) | | | | |
| | SNP | hH19DMR sF1 | AGGTTGGGGAGATGGGAGGAGATAC (SEQ ID NO.44) | 395 | 68 | | |
| | | hH19DMR sR1 | GTGGATAATGCCCGACCTGAAGATC (SEQ ID NO.45) | | | | |
| GTL2 | BS | GTL2 BSF1 | GGGTTGGGTTTTGTTAGTTGTTTGT (SEQ ID NO.46) | 459 | 57 | AL117190 | 14q32 |
| | | GTL2 BSR1 | ACAATTTAACAACAACTTTCCTCCAAA (SEQ ID NO.47) | | | | |
| DIRAS3 | BS | DIRAS3 BSF1 | TGTTGTTTTGTTTGATATTTGTTGTT (SEQ ID NO.14) | 349 | 57 | AF202543 | 1 p31 |
| | | DIRAS3 BSR1 | CCTTAAACTTCTAAACTAACCCCTC (SEQ ID NO.15) | | | | |
| | SNP | DIRAS3 SF1 | ACTTACCTTTCTCGGAGGCACG (SEQ ID NO.48) | 321 | 65 | | |
| | | DIRAS3 SR1 | AACAGTTCCTCCCCAACCTGTAAC (SEQ ID NO.49) | | | | |
| NAP1L5 | BS | NAP1L5 BS2F1 | TGATAGTGGGAAGTTAGTTAAGTGT (SEQ ID NO.16) | 348 | 57 | AC108065 | 4q21-q22 |
| | | NAP1L5 BS2R1 | AAAAATCTAAAACTCCTCAACCATC (SEQ ID NO.17) | | | | |
| | SNP | NAP1L5 SF1 | GCTGTCACAGTCTCCACCCTGC (SEQ ID NO.50) | 202 | 66 | | |
| | | NAP1L5 SR1 | CCGCATCCGCAAGATCTCTCTG (SEQ ID NO.51) | | | | |
| FAM50B | BS | FAM50B cBSF1 | GGTTTTGAGGAGAGTGTTAGGTTTT (SEQ ID NO.18) | 334 | 57 | Y18504 | 6p25.2 |
| | | FAM50B cBSR1 | AAAACTCTCTAAATAACCACAACAACTTAC (SEQ ID NO.19) | | | | |
| | SNP | FAM50B SF1 | CAGGTAATGTTCACGAGACGCCACAG (SEQ ID NO.52) | 218 | 67 | | |
| | | FAM50B SR1 | GGGGCTCCTGTTTTCACGCTGTG (SEQ ID NO.53) | | | | |
| ZAC | BS | ZAC F | GGGGTAGTYGTGTTTATAGTTTAGTA (SEQ ID NO.54) | 152 | 57 | AL109755 | 6q24-q25 |
| | | ZAC R | CRAACACCCAAACACCTACCCTA (SEQ ID NO.55) | | | | |
| GRB10 | BS | GRB10 BSF2 | GTTAGGGGTTTGYGGYGTAGAAAAT (SEQ ID NO.20) | 245 | 55 | AC004920 | 7p12.2 |
| | | GRB10 BSR2 | CCAATCCCTCRAAAACTAA (SEQ ID NO.21) | | | | |
| | SNP | GRB10 SF1 | GAACGCGCTAGCACGAAAAGC (SEQ ID NO.56) | 185 | 69 | | |
| | | GRB10SR1 | CAGTCCCTCGGAGGCTGAGTATTG (SEQ ID NO.57) | | | | |
| PEG10 | BS | PEG10 BSF1 | TTTAGTTTGGTTAGTTTAGTATTAGTATTT (SEQ ID NO.58) | 395 | 55 | AC069292 | 7q21 |
| | | PEG10 BSR1 | AAAAAATAAAATCCCACACCTAAAC (SEQ ID NO.59) | | | | |
| PEG1 | BS | hPEG1 BSF1 | AATTTTAATTATTTGATGAGTTATGAG (SEQ ID NO.60) | 275 | 57 | AB045582 | 7q32 |
| | | hPEG1 BSR1 | ATATTTTTCAAATTTCAATAACAAAC (SEQ ID NO.61) | | | | |
| | SNP | PEG1DMR sF2 | GTATCACGGTGGCGGGAGTC (SEQ ID NO.62) | 315 | 61 | | |
| | | PEG1DMR sR2 | ATGAGCGGAGACAATAAGCAAAC (SEQ ID NO.63) | | | | |
| INPP5Fv2 | BS | INPP5Fv2 BS4F1 | TAGGAATTTTAATTATAAGTTTTGTAA (SEQ ID NO.22) | 203 | 55 | AL133461 | 10q26.11 |
| | | INPP5Fv2 BS4R2 | AATACAAACAACTATTTAAACCTC (SEQ ID NO.23) | | | | |
| LIT1 | BS | LIT1 F | TTTTGGTAGGATTTTGTTGAGGAGT (SEQ ID NO.64) | 307 | 57 | U90095 | 11p15 |
| | | LIT1 R | CCTCACACCCAACCAATACCTC (SEQ ID NO.65) | | | | |
| RB1 | BS | RB1 BSF2 | GTGAAAGTGGGTTTTGGGTAGTTTG (SEQ ID NO.24) | 241 | 59 | AL392048 | 13q14.2 |
| | | RB1 BSR1 | CTTAAACATTTCCAAAACTACCCTACC (SEQ ID NO.25) | | | | |
| | SNP | RB1 SF1 | CCGCCGCCTCTACGTTTCCTTTTG (SEQ ID NO.66) | 480 | 68 | | |
| | | RB1 SR1 | CCTAGACGCTGACCATTCCCCACAAG (SEQ ID NO.67) | | | | |
| SNRPN | BS | hSNRPN BSF1 | AGGAGGTTATGGTAGTGGATTAGG (SEQ ID NO.68) | 383 | 59 | U41384 | 15q11.2 |
| | | hSNRPN BSR1 | CACCACAATAAACAAACCAAATAAC (SEQ ID NO.69) | | | | |
| | SNP | SNRPN-DMR sF3 | ACCGAGGCGAGGAGGCTATG (SEQ ID NO.70) | 335 | 68 | | |
| | | SNRPN-DMR sR3 | GACTGTGCTACTGCCCCTTCTG (SEQ ID NO.71) | | | | |
| ZNF597 | BS | ZNF597 BSF1 | GTTTTTTGATAGGAGTTGTAGAAAG (SEQ ID NO.26) | 254 | 57 | AC025283 | 16p13.3 |
| | | ZNF597 BSR1 | CAACTACCCAATAACTACAAATCCTC (SEQ ID NO.27) | | | | |
| | SNP | ZNF597 SF1 | CGGGTGGGGAATGCCTTCTTCAAG (SEQ ID NO.72) | 409 | 67 | | |
| | | ZNF597 SR1 | GAGAACTTCGACCAATCAAAGGGCAGG (SEQ ID NO.73) | | | | |
| ZNF331 | BS | ZNF331 BSF2 | GTYGGGTTTTGTTGTGTTTGTATAT (SEQ ID NO.28) | 270 | 57 | AC011487 | 19q 13.42 |
| | | ZNF331 BSR1 | ATCCCRCCACCCCCTAAAAACCAAC (SEQ ID NO.29) | | | | |
| | SNP | ZNF331 SF2 | CGTGTCAGTGTGTCCGCGTGTCA (SEQ ID NO.74) | 456 | 69 | | |
| | | ZNF331 SR2 | GGCTGCGTCACTGGTGCAAACG (SEQ ID NO.75) | | | | |
| PEG3 | BS | PEG3 BS2F3 | GGTTGTTGATTGGTTAGTATAGAAGTT (SEQ ID NO.76) | 227 | 55 | AC006115 | 19q13.4 |
| | | PEG3 BS2R0 | CTCACCTCACCTCAATACTACRCAAC (SEQ ID NO.77) | | | | |
| | SNP | PEG3-DMR sF1 | CTGTGCCCACTCTCGGACTG (SEQ ID NO.78) | 342 | 66 | | |
| | | PEG3-DMR sR1 | CACCTCGGTGCAGAAGTCTGG (SEQ ID NO.79) | | | | |
| PSIMCT-1 | BS | PSIMCT-1 BSF1 | GGATGTAGTTGGATATATTTTTTTT (SEQ ID NO.30) | 328 | 55 | AL110115 | 20q11.2 |
| | | PSIMCT-1 BSR1 | ACTTATCAAACCCTACTATTTCAAC (SEQ ID NO.31) | | | | |
| NNAT | BS | NNAT BS4F3 | GTTATGGTTTTAAGAATGGTAGGTG(SEQ ID NO.32) | 270 | 55 | AL109614 | 20q11.2-q12 |
| | | NNAT BS4R1 | AAAAAACTAAAATAAAACTCAAAAAAC (SEQ ID NO.33) | | | | |
| L3MBTL | BS | L3MBTL BSF0 | GTGTAGTTTGGAGTGAGGTTTTTTG (SEQ ID NO.34) | 331 | 55 | AL031681 | 20q13.12 |
| | | L3MBTL BSR2 | AAACCCAACTCAAAACCTAAAAAAC (SEQ ID NO.35) | | | | |
| NESPAS | BS | NESPAS BSF1 | AGTAAAGTTTTTTAGGGAGTAGTTG (SEQ ID NO.36) | 312 | 55 | AJ251760 | 20q 13.32 |
| | | NESPAS BSR1 | AACAAACTATAATAAAACTAAAAAAACTAA (SEQ ID NO.37) | | | | |
| | SNP | NESPAS SF1 | CATGGTATTTATCTGTGGGTTCAG (SEQ ID NO.80) | 287 | 62 | | |
| | | NESPAS SR1 | GCAGGGTGCTCTCTTGTTTATG (SEQ ID NO.81) | | | | |
| GNAS1A | BS | GNAS1A BSF1 | GTGTGAGTGTATTTTATTTATATGTAAGT (SEQ ID NO.38) | 236 | 55 | AF246983 | 20q13.3 |
| | | GNAS1A BSR1 | AACAAAAATCTATTTACCCTCAAAC (SEQ ID NO.39) | | | | |
| | SNP | GNAS1A SF1 | GCTGCCTTGCGTGTGAGTGC (SEQ ID NO.82) | 251 | 67 | | |
| | | GNAS1A SR1 | CGGATGGCAGGAGTCTGTTTACC (SEQ ID NO.83) | | | | |
| LINE-1 | BS | LINE-1 BSF | TTGAGTTGTGGTGGGTTTTATTTAG (SEQ ID NO.84) | 413 | 50 | X58075 | |
| | | LINE-1 BSR | TCATCTCACTAAAAAATACCAAACA (SEQ ID NO.85) | | | | |
| Alu | BS | Alu BSF | GATCTTTTTATTAAAAATATAAAAATTAGT (SEQ ID NO.86) | 152 | 43 | U14568 | |
| | | Alu BSR | GATCCCAAACTAAAATACAATAA (SEQ ID NO.87) | | | | |

The analysis by the bisulfite sequence method was carried out according to the method described in Kobayashi et al., (Hum Mol Genet. 2007; 16: 2542-51), etc. Specifically, PCR was carried out using each of the primer sets shown in Table 1, and thereafter, the obtained PCR product was purified and was then cloned into a pGEM-T vector (manufactured by Promega). The cloned sequences were each analyzed using an M13 reverse primer and Prism 3130xl Genetic Analyzer (manufactured by Applied Biosystems). On average, twenty cloned sequences were analyzed per sample.

The results of the analysis are shown in Figures 1 to 24. It became clear that, as shown in Figures 1 to 3, in genomic DNA derived from normal sperm, paternally DMRs (ZDBF2, H19, and GTL2) were in an almost completely methylated state, and as shown in Figures 4 to 22, in genomic DNA derived from normal sperm, maternally DMRs (DIRAS3, NAP1L5, FAM50B, ZAC, GRB10, PEG10, PEG1, INPP5Fv2, LIT1, RB1, SNRPN, ZNF597, ZNF331, PEG3, PSIMCT-1, NNAT, L3MBTL, NESPAS, and GNAS1A) were in an almost completely non-methylated state. In addition, it also became clear that, in genomic DNA derived from blood, paternally DMRs and maternally DMRs were both in an approximately 50% methylated state. The above results clearly show that DIRAS3, NAP1L5, FAM50B, GRB10, INPP5Fv2, RB1, ZNF597, ZNF331, PSIMCT-1, NNAT, L3MBTL, NESPAS, and GNAS1A are maternally imprinted genes.

Moreover, SNP analysis was carried out on the above described DMRs. As a result, it was found for the first time that SNPs are present in the genomic DNA derived from sperm and blood collected from normal individuals (Figures 1 to 22). Information regarding these SNPs is extremely useful when the methylated state of the above described DMRs is analyzed by the bisulfite sequence method.

### Example 3

### [Analysis using blood derived from SRS patients]

It has been known that hypomethylation of H19 is observed in patients with Russell-Silver syndrome (SRS). Moreover, it has been suggested that methylation in various other regions would be associated with SRS. In the present Example, blood was collected from each of 15 SRS patients (5 SRS patients who were born as a result of the ART and 10 SRS patients who were born as a result of natural reproduction), in whom the abnormal methylation of H19 had already been confirmed, and genomic DNA was then extracted from the collected blood. Thereafter, three types of paternally DMRs (ZDBF2, H19, and GTL2) and 19 types of maternally DMRs (DIRAS3, NAP1L5, FAM50B, ZAC, GRB10, PEG10, PEG1, INPP5Fv2, LIT1, RB1, SNRPN, ZNF597, ZNF331, PEG3, PSIMCT-1, NNAT, L3MBTL, NESPAS, and GNAS1A) were analyzed in terms of their methylated state.

The results of the analysis are shown in Figures 25 to 29, Table 2 and Tables 4 to 6. In 4 out of the 5 SRS patients who were born as a result of the ART, abnormal methylation was confirmed in maternally and paternally gDMRs, other than H19. It became clear that these 4 SRS patients exhibited a mosaic pattern in which hypermethylation was mixed with hypomethylation. On the other hand, in only 3 out of the 10 SRS patients who were born as a result of natural reproduction, abnormal methylation was confirmed in maternally and paternally gDMRs, other than H19.

Furthermore, in order to confirm whether or not the DNA abnormal methylation in these patients can be observed in a wider range, the methylation profile of the non-imprinted regions LINE1 and Alu was analyzed. Specifically, the methylated state of 28 CpG sequences comprised in a 413-bp LINE1 fragment and the methylated state of 12 CpG sequences comprised in a 152-bp Alu fragment were analyzed. As a result, as shown in Table 7, there was found no significant difference between the SRS patients who were born as a result of the ART and the SRS patients who were born as a result of natural reproduction, in terms of the methylation rates of LINE1 and Alu.

### Example 4

### [Analysis using blood derived from BWS patients]

It has been reported that hypermethylation of H19 or hypomethylation of LIT1 is observed in patients with Beckwith-Wiedemann syndrome (BWS). In the present Example, blood was collected from 7 BWS patients (1 BWS patient who was born as a result of the ART and 6 BWS patients who were born as a result of the natural reproduction), in whom the abnormal methylation of LIT1 had already been confirmed, and genomic DNA was then extracted from the collected blood. Thereafter, three types of paternally DMRs (ZDBF2, H19, and GTL2) and 19 types of maternally DMRs (DIRAS3, NAP1L5, FAM50B, ZAC, GRB10, PEG10, PEG1, INPP5Fv2, LIT1, RB1, SNRPN, ZNF597, ZNF331, PEG3, PSIMCT-1, NNAT, L3MBTL, NESPAS, and GNAS1A) were analyzed in terms of their methylated state.

The results of the analysis are shown in Figure 30 and Tables 3 to 6. In one case of BWS patient who was born as a result of the ART, abnormal hypermethylation and hypomethylation were observed in 4 DMRs. In addition, in one of the 6 BWS patients who were born as a result of the natural reproduction, abnormal methylation similar to the case of the above-mentioned BWS patient who was born as a result of the ART was observed.

From the above results, it was suggested that not only the abnormal methylation of the gDMRs of the conventionally known H19, ZDBF2, LIT1 and the like, but also the abnormal methylation of the gDMRs of novel maternally imprinted genes (DIRAS3, NAP1L5, FAM50B, GRB10, INPP5Fv2, RB1, ZNF597, ZNF331, PSIMCT-1, NNAT, L3MBTL, NESPAS, and GNAS1A), and in particular, of the gDMRs of GRB10, ZNF597, INPP5FV2, ZNF331, and FAM50B was associated with the development of an imprinting disorder such as SRS or BWS.

Table 2: DMRs whose abnormal methylation was confirmed in SRS patients

**[Table 2]**

| **Case** | **ART** | **Abnormal methylation** | | | | |
|---|---|---|---|---|---|---|
| SRS-1 | IVF-ET | H19 Hypomethylated (mosaic) | PEG1 Hypermethylated | PEG10 Hypermethylated (mosaic) | GRB10 Hypermethylated | ZNF597 Hypomethylated |
| SRS-2 | IVF-ET | H19 Hypomethylated (mosaic) | | | | |
| SRS-3 | IVF-ET | H19 Hypomethylated (mosaic) | PEG1 Hypermethylated (mosaic) | | | |
| SRS-4 | IVF-ET | H19 Hypomethylated | GRB10 Hypermethylated | | | |
| SRS-5 | IVF-ET | H19 Hypomethylated (mosaic) H19 Hypomethylated (mosaic) | INPP5F Hypermethylated | | | |
| SRS-6 | - | H19 Hypomethylated | | | | |
| SRS-7 | - | H19 Hypomethylated (mosaic) | ZNF597 Hypermethylated (mosaic) | ZNF331 Hypomethylated (mosaic) | | |
| SRS-8 | - | H19 Hypomethylated | | | | |
| SRS-9 | - | H19 Hypomethylated (mosaic) | | | | |
| SRS-10 | - | H19 Hypomethylated | | | | |
| SRS-11 | - | H19 Hypomethylated (mosaic) | PEG1 Hypermethylated | | | |
| SRS-12 | - | H19 Hypomethylated | | | | |
| SRS-13 | - | H19 Hypomethylated (mosaic) | FAM50B Hypomethylated | | | |
| SRS-14 | - | H19 Hypomethylated | | | | |
| SRS-15 | - | H19 Hypomethylated | | | | |

Table 3: DMRs whose abnormal methylation was confirmed in BWS patients

**[Table 3]**

| BWS-1 | ICSI | LIT1 Hypomethylated | ZDBF2 Hypermethylated | PEG1 Hypermethylated | NESPAS Hypomethylated (mosaic) |
|---|---|---|---|---|---|
| BWS-2 | - | LIT1 Hypomethylated | | | |
| BWS-3 | - | LIT1 Hypomethylated | | | |
| BWS-4 | - | LIT1 Hypomethylated | | | |
| BWS-5 | - | LIT1 Hypomethylated | | | |
| BWS-6 | - | LIT1 Hypomethylated | ZDBF2 Hypomethylated | ZNF331 Hypomethylated (mosaic) | |
| BWS-7 | - | LIT1 Hypomethylated | | | |

Table 4: Methylation rate of paternally DMRs (ZDBF2, H19, and IG-DMR) in SRS patients, BWS patients, and normal individuals

**[Table 4]**

| | | | | |
|---|---|---|---|---|
| Methylation rate of paternally DMRs in SRS patients | | | | |

| | | paternally DMRs | | |
|---|---|---|---|---|
| | ART | ZDBF2 | H19 | IG-DMR |
| SRS-1 | + | 455 | **21.0** | 60.9 |
| SRS-2 | + | 63.4 | **25.2** | 51.5 |
| SRS-3 | + | 45.7 | **33.7** | 52.6 |
| SRS-4 | + | 42.9 | **0.3** | 54.5 |
| SRS-5 | + | 464 | **26.2** | 536 |
| SRS-6 | - | 55.2 | **2.1** | 51.9 |
| SRS-7 | - | 43.8 | **20.3** | 51.4 |
| SRS-8 | - | 51.0 | **3.5** | 54.9 |
| SRS-9 | - | 40.2 | **25.3** | 49.2 |
| SRS-10 | - | 41.0 | **0.4** | 57.0 |
| SRS-11 | - | 43.8 | **16.3** | 60.6 |
| SRS-12 | - | 50.5 | **6.0** | 54.4 |
| SRS-13 | - | 51.8 | **34.1** | 60.2 |
| SRS-14 | - | 37.8 | **2.5** | 61.5 |
| SRS-15 | - | 50.5 | **3.3** | 53.4 |
| | | | | |
| Methylation rate of paternally DMRs in BWS patients | | | | |

| | ART | ZDBF2 | H19 | IG-DMR |
|---|---|---|---|---|
| BWS-1 | + | **93.3** | 57.3 | 52.0 |
| BWS-2 | - | 47.3 | 59.3 | 49.6 |
| BWS-3 | - | 47.6 | 41.9 | 51.9 |
| BWS-4 | - | 47.6 | 61.1 | 55.0 |
| BWS-5 | - | 49.5 | 55.2 | 47.3 |
| BWS-6 | - | **95.2** | 46.3 | 55.7 |
| BWS-7 | - | 51.0 | 51.5 | 55.8 |
| | | | | |
| Methylation rate of paternally DMRs in normal blood (n = 20) | | | | |
| | | ZDBF2 | H19 | IG-DMR |
| mean± SD | | 50.8±3.0 | 48.6±4.2 | 53.3±1.2 |
| | | | | |
| Methylation rate of paternally DMRs in normal sperm (n = 20) | | | | |

| | | ZDBF2 | H19 | IG-DMR |
|---|---|---|---|---|
| mean ± SD | | 98.2±0.9 | 96.6±1.0 | 97.3±0.4 |

Table 5: Methylation rate of maternally DMRs (DIRAS3, NAP1L5, FAM50B, ZAC, GRB10, PEG10, PEG1, INPP5Fv2, LIT1, and RB1) in SRS patients, BWS patients, and normal individuals

**[Table 5]**

| Methylation rate of maternally DMRs in SRS patients (1) | | | | | | | | | | | |
|---|---|---|---|---|---|---|---|---|---|---|---|
| | | **maternally DMRs** | | | | | | | | | |
| | **ART** | **DIRAS3** | **NAP1L5** | **FAM50B** | **ZAC** | **GRB10** | **PEG10** | **PEG1** | **INPP5F** | **LIT1** | **RB1** |
| SRS-1 | + | 45.7 | 56.5 | 41.6 | 44.5 | 97.1 | 78.8 | 99.6 | 47.7 | 54.1 | 49.2 |
| SRS-2 | + | 58.6 | 46.5 | 44.0 | 43.2 | 58.7 | 54.7 | 58.7 | 53.8 | 60.1 | 39.1 |
| SRS-3 | + | 46.6 | 56.7 | 54.2 | 44.9 | 50.0 | 49.8 | 73.8 | 58.2 | 43.9 | 46.7 |
| SRS-4 | + | 56.5 | 42.9 | 42.9 | 53.3 | 95.9 | 48.4 | 53.4 | 54.6 | 49.0 | 47.7 |
| SRS-5 | + | 49.2 | 44.1 | 41.3 | 46.3 | 55.6 | 51.0 | 63.3 | 97.3 | 53.2 | 54.9 |
| SRS-6 | - | 59.4 | 41.5 | 48.7 | 58.8 | 49.6 | 48.2 | 52.7 | 60.4 | 48.2 | 46.2 |
| SRS-7 | - | 39.4 | 41.4 | 35.7 | 53.0 | 53.8 | 56.4 | 52.4 | 59.8 | 46.4 | 62.1 |
| SRS-8 | - | 61.4 | 55.8 | 38.8 | 50.4 | 56.7 | 46.4 | 62.2 | 44.6 | 50.7 | 62.1 |
| SRS-9 | - | 56.2 | 45.5 | 39.7 | 55.7 | 49.4 | 44.8 | 54.7 | 45.7 | 42.5 | 51.0 |
| SRS-10 | - | 49.9 | 48.4 | 55.6 | 45.7 | 62.3 | 55.5 | 52.0 | 53.9 | 39.9 | 37.9 |
| SRS-11 | - | 41.3 | 40.2 | 40.3 | 53.6 | 55.7 | 46.0 | 95.2 | 56.3 | 33.9 | 57.1 |
| SRS-12 | - | 52.4 | 43.5 | 48.5 | 45.3 | 62.9 | 44.2 | 52.1 | 49.6 | 37.7 | 48.2 |
| SRS-13 | - | 46.7 | 49.3 | 0.4 | 53.4 | 59.1 | 46.7 | 40.6 | 52.9 | 55.8 | 47.8 |
| SRS-14 | - | 59.5 | 39.5 | 46.9 | 49.1 | 55.4 | 47.4 | 51.8 | 55.8 | 55.8 | 53.3 |
| SRS-15 | - | 53.4 | 45.7 | 49.2 | 49.6 | 55.4 | 45.4 | 52.0 | 58.8 | 53.6 | 35.5 |

| Methylation rate of maternally DMRs in BWS patients (1) | | | | | | | | | | | |
|---|---|---|---|---|---|---|---|---|---|---|---|
| | **ART** | **DIRAS3** | **NAP1L5** | **FAM50B** | **ZAC** | **GRB10** | **PEG10** | **PEG1** | **INPP5F** | **LIT1** | **RB1** |
| BWS-1 | + | 44.9 | 47.6 | 46.6 | 49.2 | 45.8 | 52.2 | 97.8 | 42.9 | 0.0 | 46.7 |
| BWS-2 | - | 52.2 | 59.1 | 48.3 | 50.0 | 44.4 | 51.4 | 49.8 | 53.5 | 0.0 | 47.7 |
| BWS-3 | - | 47.8 | 41.2 | 37.6 | 41.1 | 46.0 | 50.0 | 53.3 | 52.7 | 0.0 | 42.3 |
| BWS-4 | - | 52.4 | 54.0 | 43.6 | 56.0 | 52.7 | 46.0 | 51.3 | 52.7 | 0.7 | 48.7 |
| BWS-5 | - | 44.0 | 43.1 | 45.4 | 43.2 | 46.0 | 44.3 | 49.8 | 46.2 | 0.0 | 50.8 |
| BWS-6 | - | 42.8 | 44.7 | 44.6 | 46.3 | 55.4 | 51.5 | 48.4 | 54.8 | 1.3 | 46.7 |
| BWS-7 | - | 51.6 | 46.9 | 52.7 | 49.1 | 58.1 | 46.3 | 36.4 | 51.2 | 0.0 | 42.0 |
| Maternally methylation rate in normal blood (1) (n = 20) | | | | | | | | | | | |

| | **ART** | **DIRAS3** | **NAP1L5** | **FAM50B** | **ZAC** | **GRB10** | **PEG10** | **PEG1** | **INPP5F** | **LIT1** | **RB1** |
|---|---|---|---|---|---|---|---|---|---|---|---|
| mean±SD | | 52.6±5.0 | 45.8±4.1 | 53.3±5.6 | 48.4±5.9 | 53.9±2.3 | 51.6±2.5 | 49.8±5.3 | 52.2±4.1 | 53.2±3.7 | 45.9±2.3 |
| Maternally methylation rate in normal sperm (1) (n = 20) | | | | | | | | | | | |

| | **ART** | **DIRAS3** | **NAP1L5** | **FAM50B** | **ZAC** | **GRB10** | **PEG10** | **PEG1** | **INPP5F** | **LIT1** | **RB1** |
|---|---|---|---|---|---|---|---|---|---|---|---|
| mean±SD | | 0.4±0.1 | 0.7±0.8 | 1.6±0.5 | 0.5±0.6 | 0.7±1.0 | 0.3±0.4 | 0.4±0.4 | 0.3±0.2 | 0.9±0.9 | 0.6±0.7 |

Table 6: Methylation rate of maternally DMRs (SNRPN, ZNF597, ZNF331, PEG3, PSIMCT-1, NNAT, L3MBTL, NESPAS, and GNAS) in SRS patients, BWS patients, and normal individuals

**[Table 6]**

| Methylation rate of maternally DMRs in SRS patients (2) | | | | | | | | | | |
|---|---|---|---|---|---|---|---|---|---|---|
| | **ART** | **SNRPN** | **ZNF597** | **ZNF331** | **PEG3** | **PSIMCT-1** | **NNAT** | **L3MBTL** | **NESPAS** | **GNAS** |
| SRS-1 | + | 47.8 | 0.0 | 58.3 | 50.3 | 59.1 | 51.7 | 54.0 | 56.2 | 53.6 |
| SRS-2 | + | 49.7 | 37.7 | 49.4 | 51.7 | 55.8 | 60.3 | 43.3 | 46.9 | 56.8 |
| SRS-3 | + | 55.1 | 44.7 | 55.8 | 52.2 | 43.7 | 59.0 | 41.5 | 49.4 | 57.8 |
| SRS-4 | + | 49.5 | 52.8 | 56.9 | 60.2 | 51.6 | 57.1 | 46.2 | 47.5 | 59.7 |
| SRS-5 | + | 45.9 | 47.7 | 46.3 | 52.9 | 61.1 | 54.4 | 40.0 | 50.6 | 59.6 |
| SRS-6 | - | 49.7 | 41.9 | 57.7 | 51.5 | 59.7 | 49.4 | 40.3 | 39.2 | 56.8 |
| SRS-7 | - | 47.7 | **75.7** | **18.8** | 53.5 | 52.1 | 46.0 | 42.6 | 41.8 | 59.0 |
| SRS-8 | - | 45.7 | 39.3 | 43.7 | 51.1 | 54.3 | 53.2 | 32.8 | 54.7 | 52.7 |
| SRS-9 | - | 46.3 | 46.4 | 66.9 | 40.6 | 61.1 | 53.6 | 46.2 | 39.7 | 57.4 |
| SRS-10 | - | 44.5 | 39.5 | 55.0 | 43.1 | 42.8 | 55.9 | 46.9 | 37.5 | 55.3 |
| SRS-11 | - | 46.4 | 38.7 | 59.0 | 60.7 | 49.8 | 45.6 | 43.3 | 43.4 | 51.2 |
| SRS-12 | - | 59.1 | 51.6 | 57.8 | 51.4 | 62.5 | 53.8 | 41.5 | 49.6 | 58.2 |
| SRS-13 | - | 49.7 | 37.9 | 56.5 | 55.1 | 54.6 | 51.7 | 57.3 | 47.8 | 55.4 |
| SRS-14 | - | 43.6 | 35.0 | 44.0 | 51.6 | 58.7 | 55.0 | 47.4 | 36.6 | 52.0 |
| SRS-15 | - | 45.8 | 41.6 | 46.4 | 50.0 | 59.5 | 59.3 | 43.8 | 36.9 | 56.3 |

| Methylation rate of maternally DMRs in BWS patients (2) | | | | | | | | | | |
|---|---|---|---|---|---|---|---|---|---|---|
| | **ART** | **SNRPN** | **ZNF597** | **ZNF331** | **PEG3** | **PSIMCT-1** | **NNAT** | **L3MBTL** | **NESPAS** | **GNAS** |
| BWS-1 | + | 46.1 | 40.3 | 52.3 | 46.2 | 44.0 | 52.1 | 44.2 | 18.3 | 50.3 |
| BWS-2 | - | 52.9 | 41.0 | 48.1 | 49.7 | 59.2 | 60.0 | 41.9 | 43.8 | 61.6 |
| BWS-3 | - | 52.7 | 37.3 | 52.8 | 44.1 | 52.1 | 51.1 | 44.4 | 43.7 | 58.1 |
| BWS-4 | - | 52.6 | 44.4 | 42.1 | 45.6 | 53.6 | 51.0 | 45.6 | 45.0 | 56.5 |
| BWS-5 | - | 45.3 | 50.4 | 41.3 | 43.3 | 51.5 | 51.9 | 45.4 | 44.4 | 45.7 |
| BWS-6 | - | 55.0 | 44.2 | 16.8 | 51.5 | 43.5 | 50.0 | 42.0 | 39.0 | 54.8 |
| BWS-7 | - | 45.0 | 44.7 | 42.3 | 55.3 | 44.4 | 52.3 | 58.3 | 48.8 | 44.9 |

| Methylation rate of maternally DMRs in normal blood (2) (n = 20) | | | | | | | | | | |
|---|---|---|---|---|---|---|---|---|---|---|
| | **ART** | **SNRPN** | **ZNF597** | **ZNF331** | **PEG3** | **PSIMCT-1** | **NNAT** | **L3MBTL** | **NESPAS** | **GNAS** |
| mean±SD | | 49.5±5.1 | 44.0±8.2 | 56.6±7.4 | 49.9±6.1 | 51.3±3.6 | 46.3±1.0 | 41.3±8.4 | 54.0±6.5 | 55.8±4.0 |

| Methylation rate of maternally DMRs in normal sperm (2) (n = 20) | | | | | | | | | | |
|---|---|---|---|---|---|---|---|---|---|---|
| | **ART** | **SNRPN** | **ZNF597** | **ZNF331** | **PEG3** | **PSIMCT-1** | **NNAT** | **L3MBTL** | **NESPAS** | **GNAS** |
| mean±SD | | 0.5±0.6 | 0.9±0.8 | 0.7±0.5 | 0.2±0.4 | 0.6±0.5 | 0.2±0.4 | 0.6±0.7 | 0.7±0.4 | 0.8±0.3 |

Table 7: Methylation rate of non-imprinted regions (Alu and LINE1) in SRS patients, BWS patients, and normal individuals

**[Table 7]**

| Methylation rate of non-imprinted genes in SRS patients | |
|---|---|
| Non-imprinted | |
| Alu | LINE1 |
| 28.3 | 62.5 |
| 24.4 | 59.4 |
| 33.9 | 46.7 |
| 26.6 | 56.0 |
| 22.9 | 51.6 |
| 25.0 | 70.1 |
| 22.9 | 62.5 |
| 24 | 59.6 |
| 34.6 | 59.3 |
| 23.9 | 55.4 |
| 27.6 | 53.3 |
| 17.7 | 51.8 |
| 19.4 | 66.7 |
| 26.2 | 63.2 |
| 26.1 | 64.8 |

| Methylation rate of non-imprinted genes in BWS patients | |
|---|---|
| Alu | LINE1 |
| 17.7 | 54.8 |
| 28.0 | 73.8 |
| 22.2 | 59.6 |
| 26.8 | 60.5 |
| 19.2 | 57.8 |
| 26.3 | 65.2 |
| 27.6 | 64.6 |

| Methylation rate of non-imprinted genes in normal blood | |
|---|---|
| Alu | LINE1 |
| 24.1±1.3 | 60.5±3.9 |

| Methylation rate of non-imprinted genes in normal sperm | |
|---|---|
| Alu | LINE 1 |
| 21.0±5.0 | 50.5+2.3 |

## Claims

1. A method for assessing the risk that the progeny of a subject would develop an imprinting disorder, which comprises the following steps (a) and (b):
(a) a step of extracting genomic DNA from sperm collected from the subject; and
(b) a step of measuring the methylation level of cytosine residues in a CpG sequence comprised in the differentially methylation region of one or more maternally imprinted genes selected from the group consisting of DIRAS3, NAP1L5, FAM50B, GRB10, INPP5Fv2, RB1, ZNF597, ZNF331, PSIMCT-1, NNAT, L3MBTL, NESPAS, and GNAS1A, in the genomic DNA extracted in the step (a).

2. The method according to claim 1, wherein the differentially methylation region of DIRAS3 is a region at positions 1849-2197 (SEQ ID NO: 1) in the nucleotide sequence of human chromosome 1 registered under GenBank Accession No. AF202543.1 (date of update: August 13, 2001).

3. The method according to claim 1 or 2, wherein the CpG sequence comprised in the differentially methylation region of DIRAS3 is one or more CpG sequences selected from the CpG sequences at positions 1873-1874, 1893-1894, 1900-1901, 1902-1903, 1905-1906, 1912-1913, 1947-1948, 1954-1955, 1960-1961, 1991-1992, 1997-1998, 2004-2005, 2014-2015, 2021-2022, 2034-2035, 2036-2037, 2072-2073, 2074-2075, 2086-2087, 2093-2094, 2120-2121, 2128-2129, and 2150-2151, in the nucleotide sequence of human chromosome 1 registered under GenBank Accession No. AF202543.1 (date of update: August 13, 2001).

4. The method according to any one of claims 1 to 3, wherein the differentially methylation region of NAP1L5 is a region at positions 35225-35572 (SEQ ID NO: 2) in the nucleotide sequence of human chromosome 4 registered under GenBank Accession No. AC108065.3 (date of update: May 24, 2002).

5. The method according to any one of claims 1 to 4, wherein the CpG sequence comprised in the differentially methylation region of NAP1L5 is one or more CpG sequences selected from the CpG sequences at positions 35258-35259, 35260-35261, 35279-35280, 35281-35282, 35303-35304, 35308-35309, 35315-35316, 35333-35334,35342-35343,35345-35346, 35354-35355, 35369-35370, 35387-35388, 35392-35393, 35413-35414, 35435-35436, 35452-35453, 35475-35476, 35481-35482, 35491-35492, 35497-35498, 35501-35502, 35505-35506, 35507-35508, 35516-35517, 35523-35524, 35525-35526, 35531-35532, 35544-35545, and 35546-35547, in the nucleotide sequence of human chromosome 4 registered under GenBank Accession No. AC108065.3 (date of update: May 24, 2002).

6. The method according to any one of claims 1 to 4, wherein, in the differentially methylation region of NAP1L5, when the thymine residue at position 35330 in the nucleotide sequence of human chromosome 4 registered under GenBank Accession No. AC108065.3 (date of update: May 24, 2002) is mutated to an cytosine residue, the CpG sequence comprised in the differentially methylation region of NAP1L5 is one or more CpG sequences selected from the CpG sequences at positions 35258-35259, 35260-35261, 35279-35280, 35281-35282, 35303-35304, 35308-35309, 35315-35316, 35329-35330, 35333-35334, 35342-35343, 35345-35346, 35354-35355, 35369-35370, 35387-35388, 35392-35393, 35413-35414, 35435-35436, 35452-35453, 35475-35476, 35481-35482, 35491-35492, 35497-35498, 35501-35502, 35505-35506, 35507-35508, 35516-35517, 35523-35524, 35525-35526, 35531-35532, 35544-35545, and 35546-35547 in the nucleotide sequence of human chromosome 4 registered under GenBank Accession No. AC108065.3 (date of update: May 24, 2002).

7. The method according to any one of claims 1 to 6, wherein the differentially methylation region of FAM50B is a region at positions 350-683 (SEQ ID NO: 3) in the nucleotide sequence of human chromosome 6 registered under GenBank Accession No. Y18504.1 (date of update: November 14, 2006).

8. The method according to any one of claims 1 to 7, wherein the CpG sequence comprised in the differentially methylation region of FAM50B is one or more CpG sequences selected from the CpG sequences at positions 379-380, 384-385, 401-402, 419-420, 434-435, 438-439, 457-458, 471-472, 488-489, 498-499, 518-519, 539-540, 541-542, 549-550, 565-566, 582-583, 601-602, 609-610, 619-620, 643-644, and 649-650, in the nucleotide sequence of human chromosome 6 registered under GenBank Accession No. Y18504.1 (date of update: November 14, 2006).

9. The method according to any one of claims 1 to 8, wherein the differentially methylation region of GRB10 is a region at positions 42226-42470 (SEQ ID NO: 4) in the nucleotide sequence of human chromosome 7 registered under GenBank Accession No. AC004920.2 (date of update: October 15, 2003).

10. The method according to any one of claims 1 to 9, wherein the CpG sequence comprised in the differentially methylation region of GRB10 is one or more CpG sequences selected from the CpG sequences at positions 42251-42252, 42256-42257, 42264-42265, 42273-42274, 42275-42276, 42277-42278, 42284-42285, 42288-42289, 42290-42291, 42298-42299, 42305-42306, 42313-42314, 42316-42317, 42328-42329, 42335-42336, 42339-42340, 42353-42354, 42355-42356, 42361-42362, 42365-42366, 42369-42370, 42375-42376, 42388-42389, 42399-42400, 42409-42410, 42421-42422, 42423-42424, 42429-42430, 42436-42437, and 42440-42441, in the nucleotide sequence of human chromosome 7 registered under GenBank Accession No. AC004920.2 (date of update: October 15, 2003).

11. The method according to any one of claims 1 to 6, wherein the differentially methylation region of INPP5Fv2 is a region at positions 22533-22735 (SEQ ID NO: 5) in the nucleotide sequence of human chromosome 10 registered under GenBank Accession No. AL133461.10 (date of update: January 13, 2009).

12. The method according to any one of claims 1 to 11, wherein the CpG sequence comprised in the differentially methylation region of INPP5Fv2 is one or more CpG sequences selected from the CpG sequences at positions 22556-22557, 22564-22565, 22567-22568, 22571-22572, 22579-22580, 22583-22584, 22595-22596, 22601-22602, 22603-22604, 22619-22620, 22626-22627, 22628-22629, 22633-22634, 22647-22648, 22656-22657, and 22674-22675, in the nucleotide sequence of human chromosome 10 registered under GenBank Accession No. AL133461.10 (date of update: January 13, 2009).

13. The method according to any one of claims 1 to 12, wherein the differentially methylation region of RB1 is a region at positions 30347-30587 (SEQ ID NO: 6) in the nucleotide sequence of human chromosome 13 registered under GenBank Accession No. AL392048.9 (date of update: January 13, 2009).

14. The method according to any one of claims 1 to 13, wherein the CpG sequence comprised in the differentially methylation region of RB1 is one or more CpG sequences selected from the CpG sequences at positions 30370-30371, 30403-30404, 30426-30427, 30443-30444, 30452-30453, 30455-30456, 30464-30465, 30476-30477, 30479-30480, 30487-30488, 30497-30498, 30514-30515, and 30558-30559, in the nucleotide sequence of human chromosome 13 registered under GenBank Accession No. AL392048.9 (date of update: January 13, 2009).

15. The method according to any one of claims 1 to 14, wherein the differentially methylation region of ZNF597 is a region at positions 140956-141209 (SEQ ID NO: 7) in the nucleotide sequence of human chromosome 16 registered under GenBank Accession No. AC025283.6 (date of update: September 5, 2002).

16. The method according to any one of claims 1 to 15, wherein the CpG sequence comprised in the differentially methylation region of ZNF597 is one or more CpG sequences selected from the CpG sequences at positions 140981-140982, 140984-140985, 140988-140989, 140992-140993, 140997-140998, 140999-141000, 141002-141003, 141009-141010, 141014-141015, 141034-141035, 141052-141053, 141063-141064, 141073-141074, 141076-141077, 141082-141083, 141087-141088, 141103-141104, 141126-141127, 141137-141138, and 141183-141184, in the nucleotide sequence of human chromosome 16 registered under GenBank Accession No. AC025283.6 (date of update: September 5, 2002).

17. The method according to any one of claims 1 to 15, wherein, in the differentially methylation region of ZNF597, when the guanine residue at position 141064 in the nucleotide sequence of human chromosome 16 registered under GenBank Accession No. AC025283.6 (date of update: September 5, 2002) is mutated to an adenine residue, the CpG sequence comprised in the differentially methylation region of ZNF597 is one or more CpG sequences selected from the CpG sequences at positions 140981-140982, 140984-140985, 140988-140989, 140992-140993, 140997-140998, 140999-141000, 141002-141003, 141009-141010, 141014-141015, 141034-141035, 141052-141053, 141073-141074, 141076-141077, 141082-141083, 141087-141088, 141103-141104, 141126-141127, 141137-141138, and 141183-141184 in the nucleotide sequence of human chromosome 16 registered under GenBank Accession No. AC025283.6 (date of update: September 5, 2002).

18. The method according to any one of claims 1 to 17, wherein the differentially methylation region of ZNF331 is a region at positions 106090-106360 (SEQ ID NO: 8) in the nucleotide sequence of human chromosome 19 registered under GenBank Accession No. AC011487.5 (date of update: February 28, 2001).

19. The method according to any one of claims 1 to 18, wherein the CpG sequence comprised in the differentially methylation region of ZNF331 is one or more CpG sequences selected from the CpG sequences at positions 106115-106116, 106117-106118, 106123-106124, 106127-106128, 106129-106130, 106138-106139, 106142-106143, 106147-106148, 106149-106150, 106168-106169, 106173-106174, 106197-106198, 106207-106208, 106220-106221, 106225-106226, 106235-106236, 106249-106250, 106259-106260, 106275-106276, 106285-106286, 106303-106304, 106307-106308, 106313-106314, 106317-106318, 106328-106329, and 106333-106334, in the nucleotide sequence of human chromosome 19 registered under GenBank Accession No. AC011487.5 (date of update: February 28, 2001).

20. The method according to any one of claims 1 to 18, wherein, in the differentially methylation region of ZNF331, when the cytosine residue at position 106235 in the nucleotide sequence of human chromosome 19 registered under GenBank Accession No. AC011487.5 (date of update: February 28, 2001) is mutated to a thymine residue, the CpG sequence comprised in the differentially methylation region of ZNF331 is one or more CpG sequences selected from the CpG sequences at positions 106115-106116, 106117-106118, 106123-106124, 106127-106128, 106129-106130, 106138-106139, 106142-106143, 106147-106148, 106149-106150, 106168-106169, 106173-106174, 106197-106198, 106207-106208, 106220-106221, 106225-106226, 106249-106250, 106259-106260, 106275-106276, 106285-106286, 106303-106304, 106307-106308, 106313-106314, 106317-106318, 106328-106329, and 106333-106334 in the nucleotide sequence of human chromosome 19 registered under GenBank Accession No. AC011487.5 (date of update: February 28, 2001).

21. The method according to any one of claims 1 to 20, wherein the differentially methylation region of PSIMCT-1 is a region at positions 20820-21147 (SEQ ID NO: 9) in the nucleotide sequence of human chromosome 20 registered under GenBank Accession No. AL110115.38 (date of update: January 13, 2009).

22. The method according to any one of claims 1 to 21, wherein the CpG sequence comprised in the differentially methylation region of PSIMCT-1 is one or more CpG sequences selected from the CpG sequences at positions 20844-20845, 20872-20873, 20883-20884, 20892-20893, 20898-20899, 20903-20904, 20908-20909, 20919-20920, 20939-20940, 20944-20945, 20951-20952, 20953-20954, 20972-20973, 20979-20980, 20985-20986, 20995-20996, 21007-21008, 21009-21010, 21014-21015, 21018-21019, 21020-21021, 21023-21024, 21047-21048, 21064-21065, 21082-21083, and 21086-21087, in the nucleotide sequence of human chromosome 20 registered under GenBank Accession No. AL110115.38 (date of update: January 13, 2009).

23. The method according to any one of claims 1 to 22, wherein the differentially methylation region of NNAT is a region at positions 61633-61902 (SEQ ID NO: 10) in the nucleotide sequence of human chromosome 20 registered under GenBank Accession No. AL109614.28 (date of update: January 13, 2009).

24. The method according to any one of claims 1 to 23, wherein the CpG sequence comprised in the differentially methylation region of NNAT is one or more CpG sequences selected from the CpG sequences at positions 61659-61660, 61666-61667, 61708-61709, 61719-61720, 61757-61758, 61759-61760, 61765-61766, 61778-61779, 61782-61783, 61795-61796, 61797-61798, 61804-61805, 61806-61807, 61812-61813, 61820-61821, 61830-61831, 61837-61838, 61846-61847, 61853-61854, and 61870-61871, in the nucleotide sequence of human chromosome 20 registered under GenBank Accession No. AL109614.28 (date of update: January 13, 2009).

25. The method according to any one of claims 1 to 24, wherein the differentially methylation region of L3MBTL is a region at positions 161428-161758 (SEQ ID NO: 11) in the nucleotide sequence of human chromosome 20 registered under GenBank Accession No. AL031681.16 (date of update: January 13, 2009).

26. The method according to any one of claims 1 to 25, wherein the CpG sequence comprised in the differentially methylation region of L3MBTL is one or more CpG sequences selected from the CpG sequences at positions 161454-161455, 161480-161481, 161497-161498, 161517-161518, 161523-161524, 161541-161542, 161545-161546, 161552-161553, 161571-161572, 161573-161574, 161584-161585, 161592-161593, 161603-161604, 161615-161616, 161633-161634, 161640-161641, 161647-161648, 161658-161659, 161664-161665, 161670-161671, 161679-161680, 161687-161688, 161690-161691, 161700-161701, 161705-161706, 161720-161721, and 161733-161734, in the nucleotide sequence of human chromosome 20 registered under GenBank Accession No. AL031681.16 (date of update: January 13, 2009).

27. The method according to any one of claims 1 to 26, wherein the differentially methylation region of NESPAS is a region at positions 12577-12888 (SEQ ID NO: 12) in the nucleotide sequence of human chromosome 20 registered under GenBank Accession No. AJ251760.1 (date of update: November 14, 2006).

28. The method according to any one of claims 1 to 27, wherein the CpG sequence comprised in the differentially methylation region of NESPAS is one or more CpG sequences selected from the CpG sequences at positions 12615-12616, 12620-12621, 12624-12625, 12631-12632, 12639-12640, 12646-12647, 12659-12660, 12666-12667, 12668-12669, 12670-12671, 12699-12700, 12706-12707, 12719-12720, 12735-12736, 12792-12793, 12806-12807, 12828-12829, 12835-12836, 12859-12860, and 12862-12863, in the nucleotide sequence of human chromosome 20 registered under GenBank Accession No. AJ251760.1 (date of update: November 14, 2006).

29. The method according to any one of claims 1 to 28, wherein the differentially methylation region of GNAS1A is a region at positions 1698-1933 (SEQ ID NO: 13) in the nucleotide sequence of human chromosome 20 registered under GenBank Accession No. AF246983.1 (date of update: November 20, 2000).

30. The method according to any one of claims 1 to 29, wherein the CpG sequence comprised in the differentially methylation region of GNAS1A is one or more CpG sequences selected from the CpG sequences at positions 1727-1728, 1734-1735, 1737-1738, 1747-1748, 1749-1750, 1757-1758, 1762-1763, 1766-1767, 1769-1770, 1782-1783, 1785-1786, 1791-1792, 1807-1808, 1811-1812, 1815-1816, 1818-1819, 1831-1832, 1836-1837, 1842-1843, 1883-1884, and 1908-1909, in the nucleotide sequence of human chromosome 20 registered under GenBank Accession No. AF246983.1 (date of update: November 20, 2000).

31. The method according to any one of claims 1 to 30, wherein the imprinting disorder is Russell-Silver syndrome or Beckwith-Wiedemann syndrome.

32. The method according to any one of claims 1 to 31, wherein the subject is a patient with male infertility.

33. The method according to any one of claims 1 to 32, wherein, in the step (b), the methylation level is measured by a bisulfite sequence method.

34. The method according to claim 33, wherein the bisulfite sequence method uses one or more primer sets selected from the following i) to xiii):
i) a primer set for amplifying the differentially methylation region of DIRAS3, which consists of the nucleotide sequences shown in SEQ ID NOS: 14 and 15;
ii) a primer set for amplifying the differentially methylation region of NAP1L5, which consists of the nucleotide sequences shown in SEQ ID NOS: 16 and 17;
iii) a primer set for amplifying the differentially methylation region of FAM50B, which consists of the nucleotide sequences shown in SEQ ID NOS: 18 and 19;
iv) a primer set for amplifying the differentially methylation region of GRB10, which consists of the nucleotide sequences shown in SEQ ID NOS: 20 and 21;
v) a primer set for amplifying the differentially methylation region of INPP5Fv2, which consists of the nucleotide sequences shown in SEQ ID NOS: 22 and 23;
vi) a primer set for amplifying the differentially methylation region of RB1, which consists of the nucleotide sequences shown in SEQ ID NOS: 24 and 25;
vii) a primer set for amplifying the differentially methylation region of ZNF597, which consists of the nucleotide sequences shown in SEQ ID NOS: 26 and 27;
viii) a primer set for amplifying the differentially methylation region of ZNF331, which consists of the nucleotide sequences shown in SEQ ID NOS: 28 and 29;
ix) a primer set for amplifying the differentially methylation region of PSIMCT-1, which consists of the nucleotide sequences shown in SEQ ID NOS: 30 and 31;
x) a primer set for amplifying the differentially methylation region of NNAT, which consists of the nucleotide sequences shown in SEQ ID NOS: 32 and 33;
xi) a primer set for amplifying the differentially methylation region of L3MBTL, which consists of the nucleotide sequences shown in SEQ ID NOS: 34 and 35;
xii) a primer set for amplifying the differentially methylation region of NESPAS, which consists of the nucleotide sequences shown in SEQ ID NOS: 36 and 37; and
xiii) a primer set for amplifying the differentially methylation region of GNAS1A, which consists of the nucleotide sequences shown in SEQ ID NOS: 38 and 39.

35. A test kit for assessing the risk that the progeny of a subject would develop an imprinting disorder, wherein the test kit comprises one or more primer sets selected from the following i) to xiii):
i) a primer set for amplifying the differentially methylation region of DIRAS3, which consists of the nucleotide sequences shown in SEQ ID NOS: 14 and 15;
ii) a primer set for amplifying the differentially methylation region of NAP1L5, which consists of the nucleotide sequences shown in SEQ ID NOS: 16 and 17;
iii) a primer set for amplifying the differentially methylation region of FAM50B, which consists of the nucleotide sequences shown in SEQ ID NOS: 18 and 19;
iv) a primer set for amplifying the differentially methylation region of GRB10, which consists of the nucleotide sequences shown in SEQ ID NOS: 20 and 21;
v) a primer set for amplifying the differentially methylation region of INPP5Fv2, which consists of the nucleotide sequences shown in SEQ ID NOS: 22 and 23;
vi) a primer set for amplifying the differentially methylation region of RB1, which consists of the nucleotide sequences shown in SEQ ID NOS: 24 and 25;
vii) a primer set for amplifying the differentially methylation region of ZNF597, which consists of the nucleotide sequences shown in SEQ ID NOS: 26 and 27;
viii) a primer set for amplifying the differentially methylation region of ZNF331, which consists of the nucleotide sequences shown in SEQ ID NOS: 28 and 29;
ix) a primer set for amplifying the differentially methylation region of PSIMCT-1, which consists of the nucleotide sequences shown in SEQ ID NOS: 30 and 31;
x) a primer set for amplifying the differentially methylation region of NNAT, which consists of the nucleotide sequences shown in SEQ ID NOS: 32 and 33;
xi) a primer set for amplifying the differentially methylation region of L3MBTL, which consists of the nucleotide sequences shown in SEQ ID NOS: 34 and 35;
xii) a primer set for amplifying the differentially methylation region of NESPAS, which consists of the nucleotide sequences shown in SEQ ID NOS: 36 and 37; and
xiii) a primer set for amplifying the differentially methylation region of GNAS1A, which consists of the nucleotide sequences shown in SEQ ID NOS: 38 and 39.


SEQUENCE LISTING

<110>  TOHOKU UNIVERSITY

<120>  Method of evaluation of risk for imprinting disorder

<130>  P20110420

<160>  87

<170>  PatentIn version 3.5

<210>  1
<211>  349
<212>  DNA
<213>  Homo sapiens

<220>
<223>  Inventor: Arima, Takahiro

<400>  1
ccttgaactt ctggactagc ccctcgattg ttgtagatgc caagcggacc tcgcgccgct      60

ctgcgttggg ccagcccctc acagctggtt tcttaccacg tattgcgcaa gcggaatcta     120

tgcctgttac ccacactccc tgcgcccccg caccccgctc ctgtgcgcaa gtcggaatat     180

aaaaccgcgg aggagtgagc tcttggggtg tccagttggt tgccgcggca gtctctccga     240

gcagcgcatt tgtcttctag gctgcttggt tcgtgcctcc gagaaaggta agtctttctt     300

tcgctttttt aggggtactt gaaaacaaca agtgtcagac aaagcagca                 349

<210>  2
<211>  348
<212>  DNA
<213>  Homo sapiens

<400>  2
aggggtctgg ggctcctcag ccatctgacc agccgcgctg tcagggtcac cagccgcgct      60

gtcacagtct ccaccctgcg caccgccttc cgccattacc tcctctgccg ctgcctccgc     120

cgctgccgcc gcctggctag gctccgcagg cccctggttt tccgagtcgg ccatgttaga     180

ggagaagccg cagaggtcta ggagggctcc cgcagaggcc ctgcacccga gctggtccag     240

agagatcttg cggatgcggc aagtggcggt gacgtgcggc cgcgggaatg acgttggccg     300

cggccccgcc tcctgctttc gcggcacttg actggcttcc cactatca                  348

<210>  3
<211>  334
<212>  DNA
<213>  Homo sapiens

<400>  3
aaggctctct gggtggccac agcagcttgc gctgcgccca cattgcttct gcgtgtttac      60

agctgggcac gagaaggctc agcacgcacg cacagcaggt gggggcccgc cctgcccaca     120

gcgtgaaaac aggagccccg gccagccacg gctgggcagg gccagaagcg cctcctccag 180

gatcctcccc gcgctggccc gccccacagg agcaccgccc ctaccaggag cccggagctc 240

ttcccagggc ccgcctcccc gccagggggc gatccacctc cacttcctgt ttccgcagcc 300

gccctaccag gagcctggca ctctcctcag ggcc 334

<210> 4
<211> 245
<212> DNA
<213> Homo sapiens

<400> 4
gccaggggcc tgcggcgcag aaaaccgacc cggggcctcg gggccaccgc gcgccaggcg 60

aacgcgctag cacgaaaagc gggccaacgc cgcctctggg gacgccatcc gggcgagggt 120

gggatgccgc gccaccgccc gagcgtgccc gggggctccc agcgccatca ccacgcaggt 180

gcccgggggc ccctccgcgg agccggctgc cgcccggctg caatactcag cctccgaggg 240

actgg 245

<210> 5
<211> 203
<212> DNA
<213> Homo sapiens

<400> 5
ggtgcaggca gctgtttgag cctcgaggca tcgacggacg gacagacgga cggactgact 60

gacgatgccg cgctctcaca ggcctccgcc taccgcgcag cgggctgcca ctgccgcagc 120

agccgagccc aagtctgagg gcgttgtggc tatgactttc aagatttttc tcctttttgc 180

agggcttatg gttaaagttc ctg 203

<210> 6
<211> 241
<212> DNA
<213> Homo sapiens

<400> 6
gaaagtgggc cctgggcagc ctgcgagcag ctctcacctc tggccagcaa actccccgac 60

tatgccttga gggtcaaaac gtctggattt cctgatcgat gctgtcgtcg ctgtccacgg 120

agctactgtc gccgtcagag cgggaaggca cgttcaggga gtagaagcgt gggcttgcag 180

aaagggacct gttgctgcct tacatggggg ccggcagggt agtcttggaa atgcccaaga 240

t 241

<210> 7
<211> 254
<212> DNA
<213> Homo sapiens

<400> 7
gctccctgac aggagctgca gaaagcgacg cccgaccgag acgcgacgaa gaacgccacg 60

gccactgcaa aactcccacg ctctcatgct cctttacgca ggagctgcgg gaaaagccgc 120

cggaagcgac ccgccctcag ccctctccgc catcctgccc tttgattggt cgaagttctc 180

ccgaagggga agtccttctt ttgtgttgac caattgaaca ccttaaccga ggacctgtag 240

ccactgggca gctg 254


<210> 8
<211> 271
<212> DNA
<213> ZNF331

<400> 8
ggtcccgcca ccccctggag gccagcgcgc atgcgcacgc gcacccagcg gccgcagcgc 60

gcagacagca gcagagcccg acacgcatgt gcacacagac ccctacacgt atgtgcacgt 120

acccagaccc cgacacgtat gtgagcgtac acagaccccc gatacatagc gtgctcagct 180

gttgacgtgc ctgtgcgggc acacagaccc tgacgggcgt gtgcgcacgc acagatgccg 240

acacgcatgt gcagacacaa cagagcccga c 271


<210> 9
<211> 328
<212> DNA
<213> Homo sapiens

<400> 9
gcttgtcaag ccctactgtt tcagcgatcc tcattggaga ggaatcagca gtcgaaggaa 60

cagcgggcca agcgcagtcg tgacgtggcg ttcagagagc ggaagttgtc agatttcacc 120

gggccgtaaa gcgcgctggc tgtgtcatct tccggttagc gacaacggct ctgaccgtct 180

ggcaggccgc gtggcgcccg cgccggcctt cctgcagggg actccaccgg agccttgcca 240

attccgtttg tttccctgtt gtcgcccgct tcaccctgga tcatgttcaa gaagtttgat 300

gaaaaggaaa gtgtgtccaa ctgcatcc 328


<210> 10
<211> 270
<212> DNA
<213> Homo sapiens

<400> 10
agggggctaa gatggaactc aggaggcggg ggtcggtatg gaaagagcag atggattatt 60

tttttcctct cctggcgaat gaggagcgcc cccagccacc cctcctcata aacacccccc 120

aaggcgcgca tgcgcactta ggtggcgggc gggtacttaa ggcgcggcca ccgcggctgc 180

ggcagtgcgc ccaacagcgg actccgagac cagcggatct cggcaaaccc tctttctcga 240

ccacccacct accattcttg gaaccatggc                                    270


<210>  11
<211>  331
<212>  DNA
<213>  Homo sapiens

<400>  11
gtgtagcttg gagtgaggcc ccctggcgtg gagtcttgag gcctgctgag ggcgtgagct      60

ggcatgaggc gaagagaggg ccatggcacc gactccgaga tgggtcaagg acccgtacgg     120

gagtcgcaat cctcagaccc tcccgcgctc cagttccgga taagcgagta taagccgctg     180

aacatggcgg gagtggagca gcccccgagc cccgagctgc ggcaggaagg cgtgaccgaa     240

tacgaagatg gcggggcccc ggcgggagat ggcgaggcgg gcccccaaca ggcgggtagg     300

agccccgctc cccaggccct gagctgggcc c                                    331


<210>  12
<211>  312
<212>  DNA
<213>  Homo sapiens

<400>  12
ggcaggctgt agtggggcta aaggagctga ctgaccagcg ctgcggccgg tgcccgaagc      60

agcgagcagc gcacctcagc tccggttgcc gcgcggcatg gtatttatct gtgggttcag     120

tgcgcacagc gttgtccagt ctcgaaacca ccctaaaccg gcattaaacc ccaaccaaaa     180

tcccctttaa cccttcccta gaacagcagg acctgcgaaa ctctgaggcc gctttgtgag     240

gtcctcctct gcgcagcacg ccccccaccc ctctcttggt gccgccgcag ctactcccta     300

gggggctttg ct                                                         312


<210>  13
<211>  236
<212>  DNA
<213>  Homo sapiens

<400>  13
gtgtgagtgc acctcactca catgtaagtc ggggagcgcc ggggcctccc gcggaagtgc      60

gagtcgcccg gcgcaggcaa gagccgccgg gtccgggaca aggcaggggc gcccgggcgc     120

cgctgccttg ccccggggcg cctccgggca ccccccaatt ctctctcttt tctcttctcc     180

attggcgtgc ccaagagcca aaccaaggag cgcctgaggg taaacagact cctgcc         236


<210>  14
<211>  26
<212>  DNA
<213>  Artificial Sequence

<220>
<223>  DIRAS3 Forward primer

<400>  14
tgttgttttg tttgatattt gttgtt                                            26


<210>  15
<211>  25
<212>  DNA
<213>  Artificial Sequence

<220>
<223>  DIRAS3 Reverse primer

<400>  15
ccttaaactt ctaaactaac ccctc                                             25


<210>  16
<211>  25
<212>  DNA
<213>  Artificial Sequence

<220>
<223>  NAP1L5 Forward primer

<400>  16
tgatagtggg aagttagtta agtgt                                             25


<210>  17
<211>  25
<212>  DNA
<213>  Artificial Sequence

<220>
<223>  NAP1L5 Reverse primer

<400>  17
aaaaatctaa aactcctcaa ccatc                                             25


<210>  18
<211>  25
<212>  DNA
<213>  Artificial Sequence

<220>
<223>  FAM50B Forward primer

<400>  18
ggttttgagg agagtgttag gtttt                                             25


<210>  19
<211>  30
<212>  DNA
<213>  Artificial Sequence

<220>
<223>  FAM50B Reverse primer

<400>  19
aaaactctct aaataaccac aacaacttac                                        30

<210>  20
<211>  25
<212>  DNA
<213>  Artificial Sequence

<220>
<223>  GRB10 Forward primer

<400>  20
gttaggggtt tgyggygtag aaaat                                          25


<210>  21
<211>  19
<212>  DNA
<213>  Artificial Sequence

<220>
<223>  GRB10 Reverse primer

<400>  21
ccaatccctc raaaactaa                                                 19


<210>  22
<211>  27
<212>  DNA
<213>  Artificial Sequence

<220>
<223>  INPP5Fv2 Forward primer

<400>  22
taggaatttt aattataagt tttgtaa                                        27


<210>  23
<211>  24
<212>  DNA
<213>  Artificial Sequence

<220>
<223>  INPP5Fv2 Reverse primer

<400>  23
aatacaaaca actatttaaa cctc                                           24


<210>  24
<211>  25
<212>  DNA
<213>  Artificial Sequence

<220>
<223>  RB1 Forward primer

<400>  24
gtgaaagtgg gttttgggta gtttg                                          25


<210>  25

<211>  27
<212>  DNA
<213>  Artificial Sequence

<220>
<223>  RB1 Reverse primer

<400>  25
cttaaacatt tccaaaacta ccctacc                                          27


<210>  26
<211>  25
<212>  DNA
<213>  Artificial Sequence

<220>
<223>  ZNF597 Forward primer

<400>  26
gtttttgat aggagttgta gaaag                                             25


<210>  27
<211>  26
<212>  DNA
<213>  Artificial Sequence

<220>
<223>  ZNF597 Reverse primer

<400>  27
caactaccca ataactacaa atcctc                                           26


<210>  28
<211>  25
<212>  DNA
<213>  Artificial Sequence

<220>
<223>  ZNF331 Forward primer

<400>  28
gtygggtttt gttgtgtttg tatat                                            25


<210>  29
<211>  25
<212>  DNA
<213>  Artificial Sequence

<220>
<223>  ZNF331 Reverse primer

<400>  29
atcccrccac cccctaaaaa ccaac                                            25


<210>  30
<211>  25
<212>  DNA
<213>  Artificial Sequence

<220>
<223>  PSIMCT-1 Forward primer

<400>  30
ggatgtagtt ggatatattt ttttt                                          25


<210>  31
<211>  25
<212>  DNA
<213>  Artificial Sequence

<220>
<223>  PSIMCT-1 Reverse primer

<400>  31
acttatcaaa ccctactatt tcaac                                          25


<210>  32
<211>  25
<212>  DNA
<213>  Artificial Sequence

<220>
<223>  NNAT BS4F3

<400>  32
gttatggttt taagaatggt aggtg                                          25


<210>  33
<211>  27
<212>  DNA
<213>  Artificial Sequence

<220>
<223>  NNAT BS4R1

<400>  33
aaaaaactaa aataaaactc aaaaaac                                        27


<210>  34
<211>  25
<212>  DNA
<213>  Artificial Sequence

<220>
<223>  L3MBTL Forward primer

<400>  34
gtgtagtttg gagtgaggtt ttttg                                          25


<210>  35
<211>  25
<212>  DNA
<213>  Artificial Sequence

<220>
<223>  L3MBTL Reverse primer

<400>  35
aaacccaact caaaacctaa aaaac                                          25


<210>  36
<211>  25
<212>  DNA
<213>  Artificial Sequence

<220>
<223>  NESPAS Forward primer

<400>  36
agtaaagttt tttagggagt agttg                                          25


<210>  37
<211>  30
<212>  DNA
<213>  Artificial Sequence

<220>
<223>  NESPAS Reverse primer

<400>  37
aacaaactat aataaaacta aaaaaactaa                                     30


<210>  38
<211>  29
<212>  DNA
<213>  Artificial Sequence

<220>
<223>  GNAS1A Forward primer

<400>  38
gtgtgagtgt attttattta tatgtaagt                                      29


<210>  39
<211>  25
<212>  DNA
<213>  Artificial Sequence

<220>
<223>  GNAS1A Reverse primer

<400>  39
aacaaaaatc tatttaccct caaac                                          25


<210>  40
<211>  25
<212>  DNA
<213>  Artificial Sequence

<220>
<223>  ZDBF2 BSF1

<400>  40
gttttgttag ttagattgga aaata                                          25

<210>  41
<211>  28
<212>  DNA
<213>  Artificial Sequence

<220>
<223>  ZDBF2 BSR1

<400>  41
aaaataataa ttacctaaaa ataaaaac                                          28


<210>  42
<211>  25
<212>  DNA
<213>  Artificial Sequence

<220>
<223>  H19 F2

<400>  42
tatatgggta tttttggagg ttttt                                             25


<210>  43
<211>  27
<212>  DNA
<213>  Artificial Sequence

<220>
<223>  H19 R1

<400>  43
ataaatatcc tattcccaaa taacccc                                           27


<210>  44
<211>  25
<212>  DNA
<213>  Artificial Sequence

<220>
<223>  hH19DMR sF1

<400>  44
aggttgggga gatgggagga gatac                                             25


<210>  45
<211>  25
<212>  DNA
<213>  Artificial Sequence

<220>
<223>  hH19DMR sR1

<400>  45
gtggataatg cccgacctga agatc                                             25


<210>  46

<211>  25
<212>  DNA
<213>  Artificial Sequence

<220>
<223>  GTL2 BSF1

<400>  46
gggttgggtt ttgttagttg tttgt                                              25


<210>  47
<211>  27
<212>  DNA
<213>  Artificial Sequence

<220>
<223>  GTL2 BSR1

<400>  47
acaatttaac aacaactttc ctccaaa                                            27


<210>  48
<211>  22
<212>  DNA
<213>  Artificial Sequence

<220>
<223>  DIRAS3 SF1

<400>  48
acttaccttt ctcggaggca cg                                                 22


<210>  49
<211>  24
<212>  DNA
<213>  Artificial Sequence

<220>
<223>  DIRAS3 SR1

<400>  49
aacagttcct ccccaacctg taac                                               24


<210>  50
<211>  22
<212>  DNA
<213>  Artificial Sequence

<220>
<223>  NAP1L5 SF1

<400>  50
gctgtcacag tctccaccct gc                                                 22


<210>  51
<211>  22
<212>  DNA
<213>  Artificial Sequence

<220>
<223>  NAP1L5 SR1

<400>  51
ccgcatccgc aagatctctc tg                                                22


<210>  52
<211>  26
<212>  DNA
<213>  Artificial Sequence

<220>
<223>  FAM50B SF1

<400>  52
caggtaatgt tcacgagacg ccacag                                            26


<210>  53
<211>  23
<212>  DNA
<213>  Artificial Sequence

<220>
<223>  FAM50B SR1

<400>  53
ggggctcctg ttttcacgct gtg                                               23


<210>  54
<211>  26
<212>  DNA
<213>  Artificial Sequence

<220>
<223>  ZAC F

<400>  54
ggggtagtyg tgtttatagt ttagta                                            26


<210>  55
<211>  23
<212>  DNA
<213>  Artificial Sequence

<220>
<223>  ZAC R

<400>  55
craacaccca aacacctacc cta                                               23


<210>  56
<211>  21
<212>  DNA
<213>  Artificial Sequence

<220>
<223>  GRB10 SF1

<400>  56
gaacgcgcta gcacgaaaag c                                                21


<210>  57
<211>  24
<212>  DNA
<213>  Artificial Sequence

<220>
<223>  GRB10 SR1

<400>  57
cagtccctcg gaggctgagt attg                                             24


<210>  58
<211>  30
<212>  DNA
<213>  Artificial Sequence

<220>
<223>  PEG10 BSF1

<400>  58
tttagtttgg ttagtttagt attagtattt                                       30


<210>  59
<211>  25
<212>  DNA
<213>  Artificial Sequence

<220>
<223>  PEG10 BSR1

<400>  59
aaaaaataaa atcccacacc taaac                                            25


<210>  60
<211>  27
<212>  DNA
<213>  Artificial Sequence

<220>
<223>  hPEG1 BSF1

<400>  60
aattttaatt atttgatgag ttatgag                                          27


<210>  61
<211>  26
<212>  DNA
<213>  Artificial Sequence

<220>
<223>  hPEG1 BSR1

<400>  61
atatttttca aatttcaata acaaac                                           26

<210>  62
<211>  20
<212>  DNA
<213>  Artificial Sequence

<220>
<223>  PEG1DMR sF2

<400>  62
gtatcacggt ggcgggagtc                                                    20


<210>  63
<211>  23
<212>  DNA
<213>  Artificial Sequence

<220>
<223>  PEG1DMR sR2

<400>  63
atgagcggag acaataagca aac                                                23


<210>  64
<211>  25
<212>  DNA
<213>  Artificial Sequence

<220>
<223>  LIT1 F

<400>  64
ttttggtagg attttgttga ggagt                                              25


<210>  65
<211>  22
<212>  DNA
<213>  Artificial Sequence

<220>
<223>  LIT1 R

<400>  65
cctcacaccc aaccaatacc tc                                                 22


<210>  66
<211>  24
<212>  DNA
<213>  Artificial Sequence

<220>
<223>  RB1 SF1

<400>  66
ccgccgcctc tacgtttcct tttg                                               24


<210>  67

<211>  26
<212>  DNA
<213>  Artificial Sequence

<220>
<223>  RB1 SR1

<400>  67
cctagacgct gaccattccc cacaag                                          26


<210>  68
<211>  24
<212>  DNA
<213>  Artificial Sequence

<220>
<223>  hSNRPN BSF1

<400>  68
aggaggttat ggtagtggat tagg                                            24


<210>  69
<211>  25
<212>  DNA
<213>  Artificial Sequence

<220>
<223>  hSNRPN BSR1

<400>  69
caccacaata aacaaaccaa ataac                                           25


<210>  70
<211>  20
<212>  DNA
<213>  Artificial Sequence

<220>
<223>  SNRPN-DMR sF3

<400>  70
accgaggcga ggaggctatg                                                 20


<210>  71
<211>  22
<212>  DNA
<213>  Artificial Sequence

<220>
<223>  SNRPN-DMR sR3

<400>  71
gactgtgcta ctgccccttc tg                                              22


<210>  72
<211>  24
<212>  DNA
<213>  Artificial Sequence

<220>
<223>  ZNF597 SF1

<400>  72
cgggtgggga atgccttctt caag                                              24


<210>  73
<211>  27
<212>  DNA
<213>  Artificial Sequence

<220>
<223>  ZNF597 SR1

<400>  73
gagaacttcg accaatcaaa gggcagg                                           27


<210>  74
<211>  23
<212>  DNA
<213>  Artificial Sequence

<220>
<223>  ZNF331 SF2

<400>  74
cgtgtcagtg tgtccgcgtg tca                                               23


<210>  75
<211>  22
<212>  DNA
<213>  Artificial Sequence

<220>
<223>  ZNF331 SR2

<400>  75
ggctgcgtca ctggtgcaaa cg                                                22


<210>  76
<211>  27
<212>  DNA
<213>  Artificial Sequence

<220>
<223>  PEG3 BS2F3

<400>  76
ggttgttgat tggttagtat agaagtt                                           27


<210>  77
<211>  26
<212>  DNA
<213>  Artificial Sequence

<220>
<223>  PEG3 BS2R0

<400>  77
ctcacctcac ctcaatacta crcaac                                          26


<210>  78
<211>  20
<212>  DNA
<213>  Artificial Sequence

<220>
<223>  PEG3-DMR sF1

<400>  78
ctgtgcccac tctcggactg                                                 20


<210>  79
<211>  21
<212>  DNA
<213>  Artificial Sequence

<220>
<223>  PEG3-DMR sR1

<400>  79
cacctcggtg cagaagtctg g                                               21


<210>  80
<211>  24
<212>  DNA
<213>  Artificial Sequence

<220>
<223>  NESPAS SF1

<400>  80
catggtattt atctgtgggt tcag                                            24


<210>  81
<211>  22
<212>  DNA
<213>  Artificial Sequence

<220>
<223>  NESPAS SR1

<400>  81
gcagggtgct ctcttgttta tg                                              22


<210>  82
<211>  20
<212>  DNA
<213>  Artificial Sequence

<220>
<223>  GNAS1A SF1

<400>  82
gctgccttgc gtgtgagtgc                                                 20

<210>  83
<211>  23
<212>  DNA
<213>  Artificial Sequence

<220>
<223>  GNAS1A SR1

<400>  83
cggatggcag gagtctgttt acc                                                23


<210>  84
<211>  25
<212>  DNA
<213>  Artificial Sequence

<220>
<223>  LINE-1 BSF

<400>  84
ttgagttgtg gtgggtttta tttag                                              25


<210>  85
<211>  25
<212>  DNA
<213>  Artificial Sequence

<220>
<223>  LINE-1 BSR

<400>  85
tcatctcact aaaaaatacc aaaca                                              25


<210>  86
<211>  30
<212>  DNA
<213>  Artificial Sequence

<220>
<223>  Alu BSF

<400>  86
gatcttttta ttaaaaatat aaaaattagt                                         30


<210>  87
<211>  23
<212>  DNA
<213>  Artificial Sequence

<220>
<223>  Alu BSR

<400>  87
gatcccaaac taaaatacaa taa                                                23